# EUROPEAN PATENT SPECIFICATION

(11) **EP 1 224 302 B1**
(45) Date of publication and mention of the grant of the patent: **14.03.2007**
(21) Application number: 00971628.3
(22) Date of filing: 27.10.2000
(51) Int. Cl.: C12N 15/62, C12N 15/12, C12N 15/48, C12N 5/10, C07K 14/78, C07K 14/15, C07K 14/16, A61K 38/17, A61K 38/16, A61P 35/00, C12N 15/88

(54) **MODIFIED VIRAL SURFACE PROTEINS WHICH BIND TO CELLS OF TUMOR VASCULATURE**
MODIFIZIERTE VIRALE OBERFLÄCHEPROTEINE, WELCHE AN VASKULATURZELLEN EINES TUMOR BINDEN
PROTEINES VIRALES DE SURFACE MODIFIEES SE LIANT AUX CELLULES DU RESEAU VASCULAIRE TUMORAL

(30) Priority: 29.10.1999 US 429273
(43) Date of publication of application: 24.07.2002
(73) Proprietor: UNIVERSITY OF SOUTHERN CALIFORNIA, Los Angeles, CA 90033 (US)
(72) Inventor: HALL, Frederick L., Glendale, CA 91203 (US); GORDON, Erlinda Maria, Glendale, CA 91203 (US); ANDERSON, W., French, San Marino, CA 91108 (US)
(74) Representative: Schüssler, Andrea
(86) International application number: PCT/IB2000/001567
(87) International publication number: WO 2001/031036

(56) References cited:
- BLOEMENDAL H.J. ET AL.: "New strategies in anti-vascular cancer therapy" EUR. J. CLIN. INVEST., vol. 29, September 1999 (1999-09), pages 802-809, XP002290990

## Description

This invention relates to viral and non-viral vector particles, including retroviral vector particles, adenoviral vector particles, adeno-associated virus vector particles, Herpes Virus vector particles, pseudotyped viruses, and non-viral vectors having a modified, or chimeric viral surface protein, such as, for example, a chimeric viral envelope polypeptide, wherein such modified viral surface protein, such as a modified viral envelope polypeptide, includes a peptide which is a tumor vasculature targeting motif (TVTM), and specifically TVTM-5. The TVTM peptide may be placed between two consecutive amino acid residues of the viral surface protein, or may replace amino acid residues which have been removed from the viral surface protein. The term "polypeptide" as used herein means a polymer of amino acids and does not refer to any particular length of polymer. Such term also includes post-translationally modified polypeptides or proteins (*e.g*., glycosylated, acetylated, phosphorylated, etc.).

### BACKGROUND OF THE INVENTION

Neoplastic cells within solid tumors develop an intimate and complex relationship with non-neoplastic tissues, including vascular endothelial cells, stromal cells, and extracellular matrix (1), These histologic features of solid tumors, which make up greater than 90% of all human cancers, taken together with the emerging mechanisms of angiogenesis that accompany tumor growth and metastasis (1, 2), has promoted the concept of targeting of tumor vasculature as a compelling therapeutic strategy (3, 4). Accordingly, anti-angiogenic gene therapy directed against microvascular endothelial cells that have been recruited into the tumor beds has been developed (5) and employed (6, 7) with considerable success (8).

Vascular endothelial growth factor (VEGF), a selective endothelial cell mitogen (9-11) and mediator of vascular permeability (12, 13), is an important factor in driving the growth, metastasis, and angiogenesis of solid tumors (14,15). Within the tumor microenvironment, there is a reported up-regulation of both VEGF and its cognate receptor(s) on tumor vascular endothelium (16). Both oncogenic transformation and hypoxic conditions that are found in most solid tumors act synergistically to modulate VEGF expression (17,18). Moreover, recent studies of VEGF expression in tumor stromal versus tumor cells have also focused on the importance of stromal fibroblasts as a source of VEGF, and, hence, a contributor to tumor angiogenesis (19). Additionally, both FGF exposure (20) and hypoxia (21, 22) serve to up-regulate the expression of VEGF receptor(s) on endothelial cells. Thus, the VEGF/receptor complex is a highly specific marker of tumor endothelium (23, 24) which can be utilized for the targeting and/or imaging of tumor vasculature (16).

In addition to expression of angiogenic growth factors, endothelial cells within the activated microvasculature of solid tumors express αᵥ integrins, which are virtually absent from the cells of established blood vessels (25-27). Indeed, fundamental roles for inducible integrin receptors and extracellular matrix (ECM) proteins in angiogenesis are well established (28). The integrin αᵥβ₃, for example, is expressed preferentially on vascular cells during the proliferative and invasive phases of angiogenesis, and serves as a multifunctional adhesion receptor that binds to a number of ECM proteins which typically constitute a provisional matrix (including collagen, fibronectin, vitronectin, and fibrinogen) and which are reported to promote cell proliferation (29, 30). Normally undetectable in quiescent blood vessels, αᵥβ₃ becomes expressed highly upon stimulation by angiogenic growth factors or tumor cell supernatant (25). Conversely, blockade of αᵥβ₃ function by specific antibodies or peptide antagonists results in unscheduled apoptosis of proliferating endothelial cells (25), suggesting that the αᵥβ₃ receptor system provides a critical survival signal that facilitates vascular cell proliferation (28). Endothelial cell apoptosis appears to be mediated by p53 and accompanied by induction of the cell cycle inhibitor p21^{WAF1/CIPI} (28). Thus, the interaction between the inducible αᵥβ₃ adhesion receptor and the provisional ECM directly regulates growth arrest signals and promotes endothelial cell survival (28).

Growth factor and/or adhesion receptors that are selectively expressed on surface of activated endothelial cells provide an advantageous locus for targeting drugs and gene therapy vectors to angiogenic tissues. Molecular screens for high affinity targeting motifs have been developed using designated panels of mAbs (31) and random phage display peptide libraries (32). Remarkably, many of the peptide ligands isolated by random phage display technology, either (i) by panning for peptides that bind the integrins αᵥβ₁ (33,34) or αᵥβ₃ (35) *in vitro,* or (ii) by isolating peptides with homing specificity for tumor blood vessels *in vivo* (36), exhibit sequences that correspond to identifiable ligand-receptor contact points within the primary structure of fibronectin. Specifically, Asn-Gly-Arg (NGR) containing sequences identified *in vitro* and *in vivo* bear striking similarities with the 9th Type III repeat of fibronectin, while Arg-Gly-Asp (RGD) sequences correspond closely with the 10th Type III repeat of fibronectin (33, 34, 36). In terms of distinguishing these tumor vasculature targeting motifs (TVTMs), NGR motifs exhibit a greater homing ratio (tumor/control organs) than comparable RGD motifs, in addition to differential affinities for defined integrins and peptide competition kinetics, suggesting that NGR and RGD peptides bind to different cellular receptors (36). In an embodiment of the present invention. Applicants have constructed a number of NGR-bearing peptide congeners displayed within the context of Moloney murine leukemia virus (MLV) envelope "escort" proteins, incorporated these modified envelope proteins into a series of chimeric retroviral vectors, and examined the performance of these vectors in targeting and transducing activated endothelial cells.

WO-A-98/44938 discloses a viral or non-viral vector particle having a modified viral surface protein wherein the viral surface protein is modified to include a targeting polypeptide including a binding region which binds to an extracellular matrix component.

US-A-5,912,234 provides peptides having specificity for fibronectin-binding and vitronectin-binding integrins.

### SUMMARY OF THE INVENTION

The present invention is directed to a vector particle, including viral vectors, such as retroviral vectors, adenoviral vectors, adeno-associated virus vectors. Herpes Virus vectors, and pseudotyped viruses, and synthetic, vectors, such as virosomes or proteoliposomes and other non-viral vectors having a modified surface protein, wherein the surface protein, such as, for example, a viral envelope polypeptide, includes a the TVTM-5 peptide which binds to a receptor of an endothelial cell, and in particular to a receptor of a cell of a blood vessel of a tumor.

The term "vector particle", as used herein, means a particle for delivering a polynucleotide to a cell. Such particles include, but are not limited to, viral vectors, non-viral vectors, and synthetic vectors such as those mentioned hereinabove.

The present invention also is directed to producer cells for producing retroviral vector particles having an envelope polypeptide which is modified to include the TVTM-5 peptide.

The present invention also is directed to the novel TVTM-5 peptide, and to polynucleotides encoding such TVTM peptide.

The present invention also is directed to a medicament containing the vector particle, as well as the use of the vector particle in the manufacture of a medicament for treating a tumor.

### BRIEF DESCRIPTION OF THE DRAWINGS

The invention now will be described with respect to the drawings, wherein:

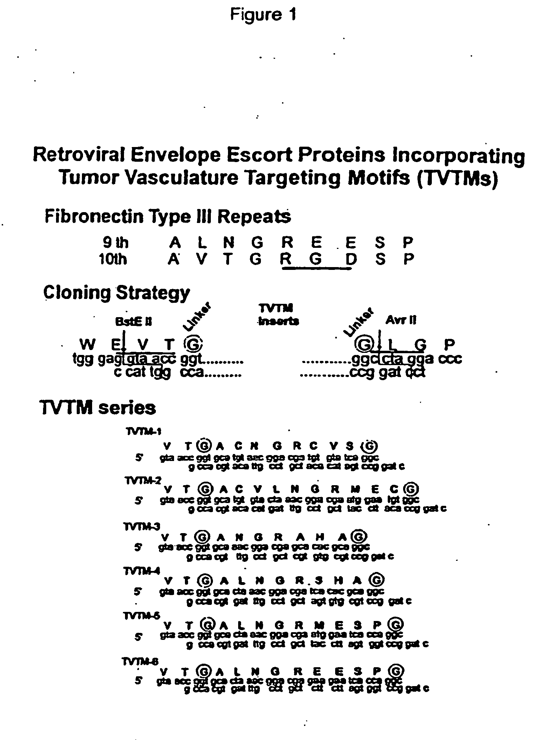

- **Fig 1.**: **Schematic diagram of tumor vasculature targeting motif (TVTM) series of retroviral escort proteins.** TVTMs were designed to target retroviral vectors to tumor vasculature. TVTM-1, TVTM-2 and TVTM-3 have been demonstrated to bind selectively to up-regulated integrins in *in vitro* panning assays, and to accumulate selectively in tumor vasculature *in vivo.* TVTM-4, TVTM-5 and TVTM-6 are novel peptides.

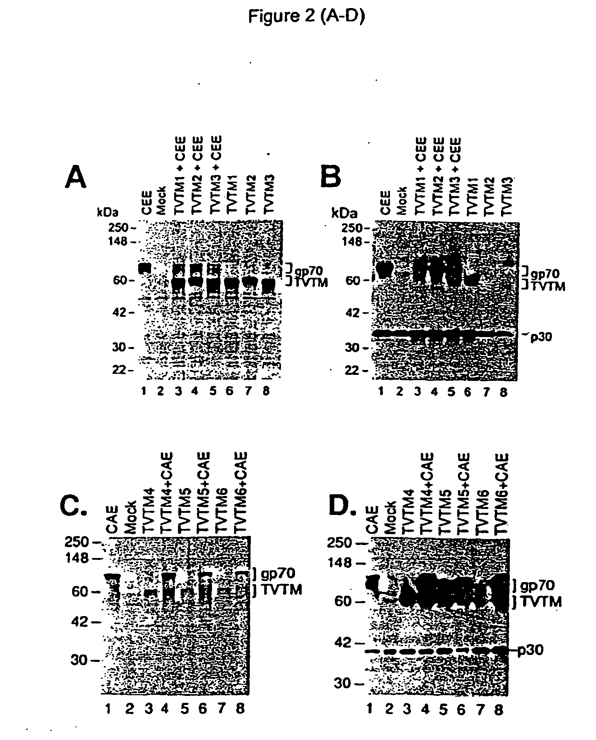

- **Fig 2.**: **Modified Envelope Protein Expression and Incorporation into Retroviral Vectors Displaying Tumor Vasculature Targeting Motifs.** A,C) Comparative *env* protein expression levels of WT CEE, WT CAE, TVTMs alone, WT CEE + TVTMs, and WT CAE + TVTMs in 293T cell lysates. B,D) Comparative levels of virion incorporation of retroviral vectors bearing WT CEE, WT CAE, TVTMs alone, WT CEE + TVTMs, and WT CAE + TVTMs. Western analysis of gp70 and gag proteins was conducted using a rat monoclonal antibody, 83A25, against the C-terminus of the gp70 env, and a polyclonal antibody against p30.

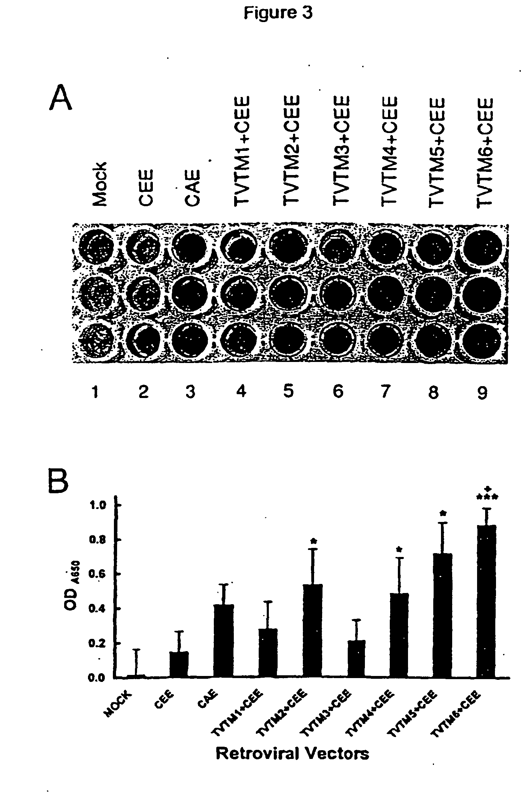

- **Fig 3.**: **KSY1 Cell Binding Affinities of Retroviral Vectors Bearing TVTM Escort proteins Co-expressed with the Ecotropic (CEE) Envelope Protein.** The comparative binding affinities of vectors bearing WT *env* (CEE* or CAE+) versus vectors displaying TVTMs to KSY-1 cells are shown as varying degrees of darkened ELISA wells (A) which are then expressed as O.D.A650 readings on a Rainbow Spectra ELISA reader (B). * = p < .05; *** = p <.001; + = p <05.

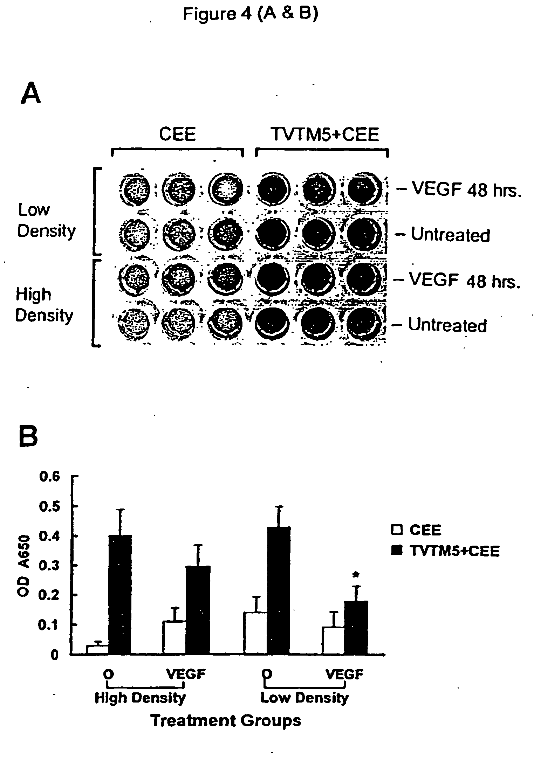

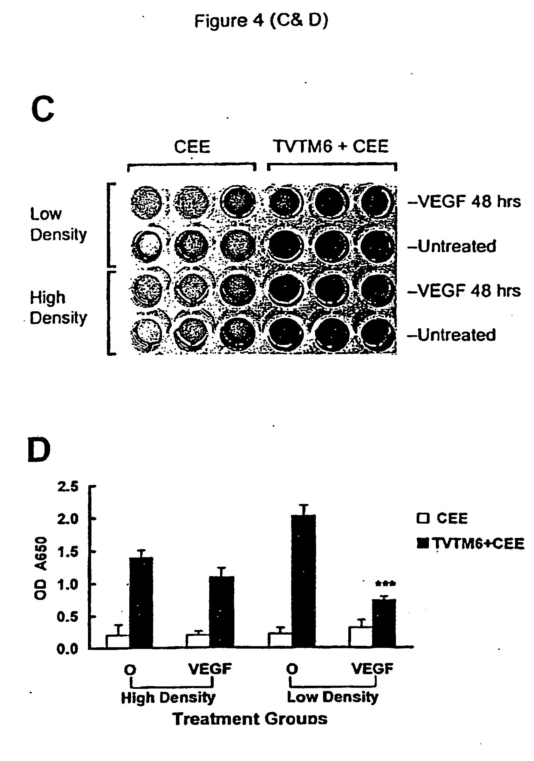

- **Fig 4.**: **Density-dependent VEGF-induced down-regulation of TVTM receptors in KSY1 cells**. The comparative binding affinities of vectors bearing WT env versus vectors displaying TVTMs to low or high density KSY1 cells following treatment with VEGF are shown as varying degrees of darkened ELISA wells (A) which are then expressed as ODA650 readings on a Rainbow Spectra ELISA reader (B). The comparative binding affinities of vectors bearing WT *env* versus vectors displaying TVTMs to low or high density HUVE cells following treatment with VEGF are shown as varying degrees of darkened ELISA wells (C) which are then expressed as O.D.A650 readings on a Rainbow Spectra ELISA reader (D). *p<.05 compared to no VEGF; *** p <.001 compared to no VEGF.

### DETAILED DESCRIPTION OF THE INVENTION

In accordance with an aspect of the present invention, there is provided a vector particle having a modified viral surface protein, such as, for example, a modified viral envelope polypeptide, for targeting the vector particles to endothelial cells, such as, for example, cells of blood vessels of tumors. The viral surface protein is modified to include the TVTM-5 peptide.

The claimed TVTM-5, has the following sequence whereas TVTM-1, TVTM-2, TVTM-3, TVTM-4, and TVTM-5 do not belong to the present invention but are shown for illustrative reasons.
TVTM-1:
↑
Ala Cys Asn Gly Arg Cys Val Ser
(SEQ ID NO:1)
TVTM-2:
Ala Cys Val Leu Asn Gly Arg Met Glu Cys
(SEQ ID NO:2)
TVTM.3:
Ala Asn Gly Arg Ala His Ala
(SEQ ID NO:3)
TVTM-4 :
Ala Leu Asn Gly Arg Ser His Ala
(SEQ ID NO:4)
TVTM-5:
Ala Leu Asn Gly Arg Met Glu Ser Pro
(SEQ ID NO: 5)
TVTM-6:
Ala Leu Asn Gly Arg Glu Glu Ser Pro
(SEQ ID NO: 6)

Such peptides and polynucleotides encoding such peptides are shown in Figure 1.

The peptides TVTM-4, TVTM-5, and TVTM-6 and polynucleotides encoding such peptides are novel, and Applicants have discovered that such peptides bind to cells of the tumor vasculature, *i.e*., such peptides bind to cells of blood vessels found in tumors.

Vector particles which have a modified surface protein including the TVTM peptide of the invention ("the TVTM peptide.") include any viral or non-viral vector particle which may be employed for gene transfer to cells *in vivo, ex vivo,* or *in vitro,* or for gene therapy. Such vector particles include, but are not limited to, retroviral vector particles, adenoviral vector particles, adenc-associated virus particles, Herpes Virus particles, pseudotyped viruses, and non-viral vectors. The TVTM peptide may be placed in any region of any viral surface protein. The TVTM peptide, in one embodiment, may be placed between two consecutive amino acid residues of a viral surface protein. Alternatively, amino acid residues of a viral surface protein are removed and replaced with the TVTM peptide.

Viral surface proteins which may be modified include, but are not limited to retroviral envelope proteins, adenoviral hexon proteins, adenoviral fiber proteins, adenoviral penton, adeno-associated virus capsid proteins and Herpes Virus envelope proteins. It is to be understood, however, that the scope of the present invention is not to be limited to any particular modified viral surface protein.

In one embodiment, the vector particle is a viral vector particle, and in one embodiment, the viral vector particle is a retroviral vector particle. Any portion of the retroviral envelope may be modified to include the targeted polypeptide. In one embodiment, the receptor binding region of the retroviral envelope is modified to include the TVTM peptide. In a further preferred embodiment one copy of the TVTM peptide is included in the viral surface protein.

In one embodiment, the TVTM peptide is inserted between two consecutively numbered amino acid residues of the native (*i.e.,* unmodified) receptor binding region of the retroviral envelope. In a preferred embodiment the viral surface protein includes the TVTM peptide near the N-terminus of the viral surface protein. Preferredly, the site into which the TVTM peptide is inserted is located at a position no more than 20 amino acids from the N-terminus of the retroviral envelope, preferably no more than 10 amino acids and most preferably between amino acids 6 and 7 from the N-terminus of the retroviral envelope. The TVTM peptide may be flanked by a linker, preferredly by a glycine linker.

In a preferred embodiment the retroviral envelope protein is an amphotropic envelope protein.

In another embodiment, amino acid residues of the receptor binding region may be removed and replaced with the TVTM peptide.

The modified surface proteins of the invention may be incorporated into the vector particles in addition to a wild-type envelope protein ("dual envelope configuration", see for example "Example 1"), or in a "single envelope configuration", i.e. in the absence of a wild-type envelope protein (see for example "Example 2"). If the dual envelope configuration is chosen, it may be advantageous to replace portions or the entire receptor-binding domain of the wild-type envelope protein with the TVTM peptide ("envelope escort proteins", see Example 1). e.g. in order to avoid stearic hindrances between envelope proteins present on the surface of the vector particle.

Accordingly, in one embodiment, there is provided a retroviral vector including a first retroviral envelope protein and at least one modified retroviral envelope protein. The first retroviral envelope protein includes a surface protein. The surface protein includes (i) a receptor binding region; (ii) a hypervariable polyproline, or "hinge" region, and (iii) a body portion. The modified retroviral envelope protein, prior to modification, includes a surface protein which includes (i) a receptor binding region; (ii) a hypervariable polyproline, or "hinge" region; and (iii) a body portion. The modified retroviral envelope protein may be modified such that at least 90% of the amino acid residues of the receptor binding region of the surface protein of the modified retroviral envelope protein have been removed and replaced with the TVTM peptide,

In one embodiment, at least 92% of the amino acid residues of the receptor binding region of the surface protein of the modified retroviral envelope protein have been removed and replaced with the TVTM peptide. In another embodiment, all of the amino acid residues of the receptor binding region of the surface protein of the modified retroviral envelope protein have been removed and replaced with the TVTM peptide.

In yet another embodiment, at least 90% of the amino acid residues of the receptor binding region of the surface protein of the modified retroviral envelope protein have been removed and replaced with the TVTM peptide, and at least a portion of the amino acid residues of the hypervariable polyproline region of the surface protein of the modified retroviral envelope protein have been removed and replaced with the TVTM peptide. In one embodiment, all of the amino acid residues of the hypervariabale polyproline region of the modified retroviral envelope protein have been removed.

In a further embodiment, the receptor binding region(s) of the modified retroviral envelope protein(s), prior to modification thereof, has (have) the sequence (SEQ ID NO:7). In the modified retroviral envelope protein(s), amino acid residues 19 through 229 of (SEQ ID NO:7) have been removed and replaced with a non-retroviral protein or peptide. In one embodiment, amino acid residues 19 through 229 of (SEQ ID NO:7) and at least a portion of the amino acid residues of the hypervariable polyproline region of the surface protein of the modified retroviral envelope protein(s) have been removed and replaced with the TVTM peptide.

In general, retroviral envelope protein(s) include a surface (SU) domain, or surface protein, and a transmembrane (TM) domain or protein. In general, the surface protein includes, in an N-terminal to C-terminal direction, the following regions: (i) a receptor binding region; (ii) a hypervariable polyproline region; and (iii) a body portion, which is associated with the transmembrane domain.

The first retroviral envelope protein includes the surface domain and the transmembrane domain. In general, such envelope protein is free of non-retroviral peptides. The first retroviral envelope protein, in one embodiment, may include regions of different tropisms. For example, in one embodiment, the first retroviral envelope protein may include a surface protein which includes (i) an ecotropic receptor binding region; (ii) an amphotropic hypervariable polyproline region; and (iii) an ecotropic body portion.

As hereinabove stated, the modified retroviral envelope protein may be a retroviral envelope protein which is modified such that at least 90% of the amino acid residues of the receptor binding region of the surface protein have been removed and replaced with the TVTM peptide. Shown in (SEQ ID NO:7) is the receptor binding region of the ecotropic envelope of Moloney Murine Leukemia Virus. The TVFM peptide may serve as an "escort" protein which provides one or more additional functions to the retroviral vector, such as, for example, "targeting" the retroviral vector to a desired target molecule on a cell found in the blood vessel of a tumor. Such retroviral vectors, while possessing such additional functions, retain the infectivity of wild-type retroviruses.

In one embodiment, the modified retroviral envelope protein(s), prior to the modification of at least the receptor binding region to include the TVTM peptide, may be an envelope which includes regions of different tropisms. For example, the modified retroviral envelope protein(s) may be a Moloney Murine Leukemia Virus envelope protein(s) which includes a surface protein (also known as gp 70 protein) having an ecotropic portion and an amphotropic portion and/or xenotropic portion.

In another embodiment, the modified retroviral envelope protein, prior to modification thereof, has a gp 70 protein which includes: (i) an ecotropic receptor binding region, i.e., (SEQ ID NO:7); (ii) an amphotropic hypervariable polyproline region. (SEQ ID NO:8); and (iii) an ecotropic body portion. At least 90% of the amino acid residues of the ecotropic receptor binding region (SEQ ID NO:7) have been removed and replaced as hereinabove described, with the TVTM peptide: In a further embodiment, at least a portion of the amphotropic hypervariable polyproline region (SEQ ID NO:8) have been removed as well. In one embodiment, amino acid residues 1 through 35 of (SEQ ID NO:8) have been removed. In another embodiment, amino acid residues I through 48 of (SEQ ID NO:8) have been removed. In yet another embodiment, all 60 amino acid residues of (SEQ ID NO:8) have been removed.

In accordance with another aspect of the present invention, there is provided a modified polynucleotide encoding a modified retroviral, envelope polypeptide (ie., the modified retroviral envelope or "escort" protein hereinabove described). The retroviral envelope polypeptide includes a receptor binding region. In the modified polynucleotide, a polynucleotide encoding at least 90% of the amino acid residues of the receptor binding region has been removed and replaced with a polynucleotide encoding the TVTM peptide, as hereinabove described.

In another preferred embodiment one copy of the polynucleotide encoding the TVTM peptide is included in the modified polynucleotide encoding the viral surface protein. In a further preferred embodiment the modified polynucleotide encoding the viral surface protein includes the polynucleotide encoding the TVTM peptide near the 5'-terminus. Preferredly, the site into which the polynucleotide encoding the TVTM peptide is inserted is located at a position no more than 20 codons from the N-terminus of the retroviral envelope, preferably no more than 10 codons and most preferably between the 6th and 7th codon from the 5'-terminus of the polynucleotide encoding the retroviral envelope.

In one embodiment, prior to modification, the polynucleotide encoding the receptor binding region encodes the sequence of (SEQ ID NO:7). In the modified polynucleotide, a polynucleotide including the codons encoding amino acid residues 19 through 229 of (SEQ ID NO:7) has been removed and replaced with the polynucleotide encoding the TVTM peptide. In another embodiment, a polynucleotide encoding at least a portion of the hypervariable polyproline region also has been removed as well. In one embodiment, the hypervariable polyproline region has the sequence (SEQ ID NO:8). The receptor binding region having the sequence (SEQ ID NO:7) is encoded by the polynucleotide having (SEQ ID NO:9) or a degenerative derivative or analogue thereof. The hypervariable polyproline region having the sequence (SEQ ID NO:8) is encoded by the polynucleotide having (SEQ ID NO:10) or a degenerative derivative or analogue thereof.

The term "derivative or analogue thereof "as used herein means that the polynucleotides encoding the polypeptides (SEQ ID NO:7) and (SEQ ID NO:8) may have sequences different from the polynucleotides (SEQ ID NO:9) and SEQ ID NO:10), yet encode the same polypeptide. Such differences in polynucleotide sequences may, for example, be due to the degeneracy of the genetic code. It is also contemplated within the scope of the present invention that, prior to the modification of (SEQ ID NO:8) or (SEQ ID NO:10) with a polynucleotide encoding a TVTM peptide. (SEQ ID NO:8) or (SEQ ID NO:10) may be modified such that one or more codons encode different amino acid residues than the unmodified sequences. Such modifications may facilitate the insertion of the polynucleotide encoding the ligand.

The above polynucleotides may be constructed by genetic engineering techniques known to those skilled in the art. For example, a first expression plasmid may be constructed which includes a polynucleotide encoding the unmodified envelope protein. The plasmid then is engineered such that a polynucleotide encoding at least 90% of the amino acid residues of the receptor binding region, and which, in some embodiments, also may encode at least a portion of the hypervariable polyproline region, has been removed, whereby such polynucleotide has been replaced with a polynucleotide encoding the TVTM peptide. The polynucleotide encoding the TVTM peptide may be contained in a second expression plasmid or may exist as a naked polynucleotide sequence. The polynucleotide encoding the TVTM peptide or the plasmid containing such polynucleotide is cut at appropriate restriction enzyme sites and cloned into the first expression plasmid which also has been cut at appropriate restriction enzyme sites. The resulting expression plasmid thus includes a polynucleotide which includes the modified retroviral envelope protein. Such plasmid also includes a polynucleotide encoding a minimal signal peptide of the retroviral envelope protein.

The term "polynucleotide" as used herein means a polymeric form of nucleotide of any length, and includes ribonucleotides and deoxyribonucleotides. Such term also includes single- and double-stranded DNA, as well as single- and double-stranded RNA. The term also includes modified polynucleotides such as methylated or capped polynucleotides.

In a preferred embodiment, the retroviral vector particle having a first envelope protein and a modified envelope protein in accordance with the present invention includes a polynucleotide encoding an agent which is capable of providing for the inhibition, prevention, or destruction of cells of the blood vessels of tumors.

It is to be understood, however, that the scope of the present invention is not to be limited to any particular agent.

The polynucleotide encoding the agent is under the control of a suitable promoter. It is to be understood, however, that the scope of the present invention is not to be limited to specific foreign genes or promoters.

The polynucleotide encoding the agent may be placed into an appropriate retroviral plasmid vector by genetic engineering techniques known to those skilled in the art.

In one embodiment, the retroviral plasmid vector may be derived from Moloney Murine Leukemia Virus and is of the LN series of vectors, which are described further in Bender, *et al*., J. Virol., Vol. 61, pgs. 1639-1649 (1987) and Miller, *et al.,* Biotechniques, Vol. 7, pgs 980-990 (1989). Such vectors have a portion of the packaging signal derived from a mouse sarcoma virus, and a mutated gag initiation codon. The term "mutated" as used herein means that the gag initiation codon has been deleted or altered such that the gag protein or fragments or truncations thereof, are not expressed.

In another embodiment, the retroviral plasmid vector may include at least four cloning, or restriction enzyme recognition sites, wherein at least two of the sites have an average frequency of appearance in eukaryotic genes of less than once in 10,000 base pairs; i.e., the restriction product has an average DNA size of at least 10,000 base pairs. Preferred cloning sites are selected from the group consisting of NotI, SnaBI, SAlI, and XhoI. In a preferred embodiment, the retroviral plasmid vector includes each of these cloning sites. Such vectors are further described in U.S. Patent No. 5,672,510, which is incorporated herein by reference in its entirety.

When a retroviral plasmid vector including such cloning sites is employed, there may also be provided a shuttle cloning vector which includes at least two cloning sites which are compatible with at least two cloning sites selected from the group consisting of NotI, SnaBI, SalI, and XhoI located on the retroviral plasmid vector. The shuttle cloning vector also includes at least one desired polynucleotide encoding the agent which is capable of being transferred from the shuttle cloning vector to the retroviral plasmid vector.

The shuttle cloning vector may be constructed from a basic "backbone" vector or fragment to which are ligated one or more linkers which include cloning or restriction enzyme recognition sites. Included in the cloning sites are the compatible, or complementary cloning sites hereinabove described. Genes and/or promoters having ends corresponding to the restriction sites of the shuttle vector may be ligated into the shuttle vector through techniques known in the art.

The shuttle cloning vector can be employed to amplify DNA sequences in prokaryotic systems. The shuttle cloning vector may be prepared from plasmids generally used in prokaryotic systems and in particular in bacteria. Thus, for example, the shuttle cloning vector may be derived from plasmids such as pBR322; pUC 18; etc.

The retroviral plasmid vector includes one or more promoters for the genes contained in the vector. Suitable promoters which may be employed include, but are not limited to, the retroviral LTR; the SV40 promoter; and the human cytomegalovirus. (CMV) promoter described in Miller, *et al.,* Biotechniques, Vol. 7, No. 9, 980-990 (1989), or any other promoter (e.g., cellular promoters such as eukaryotic cellular promoters including, but not limited to, the histone, pol III, and β-actin promoters). Other viral promoters which may be employed include, but are not limited to, adenovirus promoters, TK promoters, and B19 parvovirus promoters. The selection of a suitable promoter will be apparent to those skilled in the art from the teachings contained herein.

In one embodiment, the polynucleotide encoding the modified retroviral envelope protein is contained in a separate expression vehicle, such as an expression plasmid. Alternatively, the polynucleotide encoding the modified retroviral envelope protein may be contained in a retroviral plasmid vector for transduction and expression of the modified retroviral envelope protein in producer cell lines.

In one embodiment, the retroviral plasmid vector which includes a polynucleotide encoding the agent, and the expression vehicle including the polynucleotide encoding the modified retroviral envelope protein in accordance with the invention are transduced into a packaging cell line including nucleic acid sequences encoding the gag, pol, and wild-type (i.e., unmodified) env retroviral proteins. Examples of such packaging cell lines include, but are not limited to, the PE501, PA317 (ATCC No. CRL 9078). ψ-2, ψ-AM, PA12. T19-14X, VT-19-17-H2, ψCRE, ψCRIP, GP+E-86, GP+envAm12, and DAN cell lines as described in Miller, Human Gene Therapy, Vol. 1, pgs. 5-14 (1990), which is incorporated herein by reference in its entirety, or the 293T cell line (U.S. Patent No. 5,952,225). The vector may transduce the packaging cells through any means known in the art. Such means include, but are not limited to, electroporation, and use of liposomes, such as hereinabove described, and CaPO₄ precipitation. Such producer cells generate infectious retroviral vector particles which include the first, or unmodified wild-type retroviral envelope protein, the modified retroviral envelope protein, and a polynucleotide encoding the agent.

In another embodiment, there is provided a packaging cell which includes polynucleotides encoding the gag and pol proteins, a polynucleotide encoding a first retroviral envelope protein free of non-retroviral peptides (which in one embodiment, may be a wild-type retroviral envelope protein), and a polynucleotide encoding the modified retroviral envelope protein. A producer cell for generating retroviral vector particles which include the first and modified envelope proteins is produced by introducing into such packaging cell either a retroviral vector particle or a retroviral plasmid vector, in each case including a polynucleotide encoding the agent. The producer cell line thus generates' infectious retroviral vector particles including the first retroviral envelope protein and the modified retroviral envelope protein and the polynucleotide encoding the agent.

The retroviral vector particles, which include the first retroviral envelope protein and the modified retroviral envelope protein, and a polynucleotide encoding the agent, may be administered to a host in order to express the agent in the host. In one embodiment, the retroviral vector particles are administered to the host in an amount effective to inhibit, prevent, or destroy the growth of cells in the blood vessels in a tumor. The host may be a mammalian host, which may be a human or non-human primate host. The retroviral vector particles, upon administration to the host, travel to and transduce the cells of blood vessels of a tumor, whereby the transduced cells express the agent *in vivo.* The exact dosage of retroviral vector particles which may be administered is dependent upon a variety of factors, including the age, sex, and weight of the patient, the cells which are to be transduced, the agent which is to be administered, and the type and size of the tumor to be treated.

Accordingly, in one aspect of the present invention a medicament containing the vector particle of the invention is contemplated. In particular, the use of the vector particle of the invention in the manufacture of a medicament for the treatment a tumor is provided. Further, the use of the vector particle of the invention in the manufacture of a medicament useful to inhibit, prevent, or destroy the growth of cells of blood vessels of a tumor is provided.

The retroviral vector particles may be administered systemically, such as, for example, by intravascular administration, including intravenous and intraarterial administration.

Cells which may be transduced with the retroviral vector particles of the present invention include, but are not limited to, vascular cells found within the vasculature of tumors. Such cells include, but are not limited to, endothelial cells found in the blood vessels of tumors.

In addition, retroviral vector particles which include the modified retroviral envelope protein hereinabove described wherein said modified retroviral envelope protein includes a TVTM peptide are employed in the treatment of tumors, including malignant and non-malignant tumors. By targeting retroviral vector particles to cells of the blood vessels of tumors, the retroviral vector particles infect such cells. The tumors include, but are not limited to, all solid tumors, including carcinomas; sarcomas, including Kaposi's sarcoma, osteosarcoma and soft tissue sarcoma; colon cancer, ovarian cancer, lung cancer, brain tumors, hemangiomas, endotheliomas, hemangiosarcomas, and hepatocellular carcinoma. For example, a retroviral vector particle, including the modified retroviral envelope protein as hereinabove described and which includes the TVTM peptide, and a polynucleotide encoding a negative selective marker or "suicide" gene, such as, for example, the Herpes Simplex Virus thymidine kinase (TK) gene, or the cytosine deaminase gene may be administered to a patient, whereby the retroviral vector particles transduce the cells of the blood vessels of a tumor. After the cells are transduced with the retroviral vector particles, an interaction agent or prodrug, such as gancyclovir or acyclovir, is administered to the patient, whereby the transduced cells of the blood vessels of a tumor are killed. Thus, the blood supply to the tumor is ended, and growth of the tumor is inhibited, prevented, or destroyed. It is to be understood, however, that the scope of the present invention is not to be limited to the treatment of any particular tumor.

The retroviral vectors also may be employed to deliver polynucleotides encoding therapeutic agents for the treatment of ischemias, whereby the retroviral vectors transduce ischemic cells. Ischemias which may be treated include, but are not limited to, myocardial ischemia, renal ischemia, necrotizing enterocolitis, and stroke. It is to be understood, however, that the scope of the present invention is not intended to be limited to the treatment of any particular ischemia.

The retroviral vectors may be employed to deliver polynucleotides encoding anti-angiogenic agents to sites of active angiogenesis. Thus, such vectors may be employed in the treatment of diabetic retinopathy and corneal neovascularization, for example.

The retroviral vector particles, which include the first retroviral envelope protein and the modified retroviral envelope protein hereinabove described and a polynucleotide encoding an agent, may be administered to an animal *in vivo* as part of an animal model for the study of the effectiveness of a gene therapy treatment. The retroviral vector particles may be administered in varying doses to different animals of the same species, whereby the retroviral vector particles will transduce the desired cells of blood vessels of a tumor in the animal, or to cells located at sites of ischemia or active angiogenesis. The animals then are evaluated for the expression of the desired agent *in vivo* in the animal. From the data obtained from such evaluations, one may determine the amount of retroviral vector particles to be administered to a human patient.

In addition, the "escort" protein which forms the modified retroviral envelope protein may be employed to form proteoliposomes ; i.e., the "escort" protein forms a portion of the liposome wall. Such proteoliposomes may be employed for gene transfer or for drug delivery to desired cells of the blood vessel of a tumor, or to sites of ischemia or active angiogenesis.

Alternatively, the modified retroviral vector may include only the modified envelope protein which includes the TVTM peptide ("single envelope configuration). Such a modified envelope may be constructed as hereinabove described and further exemplified in Example 2. Generally retroviral vectors including such a modified envelope protein as the only envelope protein may be generated by techniques known to the skilled in the art from pre-packaging and packaging cells such as those hereinabove described. The TVTM peptide provides one or more additional functions to the retroviral vector, such as, for example, "targeting" the retroviral vector to a desired target molecule on a cell found in the blood vessel of a tumor. Such retroviral vectors, while possessing such additional functions, retain infectivity comparable to that of wild-type retroviruses.

The TVTM peptides also may be conjugated to agents which inhibit, prevent, or, destroy the growth of cells of blood vessels of tumors, such as endothelial cells of such blood vessels. The TVTM peptide - agent conjugate then may be administered systemically to an animal host, including mammalian, hosts, including human and non-human primate hosts: The conjugate will travel to the cells of blood vessels of a tumor, whereby the TVTM peptide will bind to the cells and deliver the agent to the cells. The TVTM peptides also may be conjugated to anti-ischemic agents or anti-angiogenic agents. The conjugate then may be administered systemically to a host, whereby the TVTM peptide will travel to an ischemic site or site of angiogenesis, whereby the TVTM peptide will bind to cells at such site to deliver an anti-ischemic agent or anti-angiogenic agent to such site.

In another embodiment, the TVTM peptide may be included as part of an adenoviral vector wherein the fiber protein of the adenoviral vector has been modified to include a TVTM peptide. Examples of such adenoviral vectors are described in U.S. Patent Nos. 5,543,328 and 5,756,086.

### EXAMPLE 1

The invention now will be described with respect to the following example. It is to be understood, however, that the scope of the present invention is not intended to be limited thereby.

### MATERIALS AND METHODS

**Molecular cloning of MLV-based escort proteins displaying tumor vasculature targeting motifs** (see Figure 1). TVTM inserts with cohesive ends were cloned into the CEE+ (ecotropic)-delta hinge envelope (*env*) construct (37), designated CEEC, which was modified from CEE+ by substitution of an amphotropic proline rich hinge region (PRR) containing three unique restriction sites (AvrII, Pst1, Stu1) and an NgoM1 restriction site (37). The MLV-based *env* construct was cut with BstEII and AvrII and the linearized *env* plasmid was verified by restriction analysis on agarose gels and purified by the Gene Clean method (Bio 101, Vista, CA) prior to ligation with the respective TVTM insert and T4 DNA Ligase (New England Biolabs, Beverly, MA) for either 3 hours at RT or overnight at 4°C. In the resulting "escort" constructs, a TVTM peptide flanked by glycine linkers replaced the entire receptor binding region of the MLV ecotropic *env* surface (SU) protein, between the BstEII site at the amino terminus and the AvrII site located proximal to the transmembrane (TM) domain. After ligation, the various constructs of plasmid DNA were transformed into XL1 Blue strain of E. coli and grown on LB agar plates under ampicillin selection. Plasmid DNA was extracted from selected transformed clones using QIAprep Miniprep Kits (Qiagen. Valencia, CA). Each construct was confirmed by enzyme digestion and analysis of the respective inserts, followed by direct DNA sequence analysis using the T7 Sequenase sequencing kit (Amersham Life Science, Inc., Cleveland, Ohio).

**Generation of retroviral vector stocks**. Retroviral vectors bearing WT *env* and/or TVTM-bearing "escort" protein constructs were assembled using a three- or four-plasmid transient transfection system (38) in which the wild type (WT) amphotropic or ecotropic env protein was co-expressed. The packaging components *gag-pol,* the WT env, the chimeric *env*, and a retroviral vector bearing a nuclear-targeted β-galactosidase expression construct expressed from CMV promoters were placed on separate plasmids, each containing the SV40 origin of replication. Ten µg of each plasmid (pcgp, either pCAE, pCEE+ or pCEEC, pESCORT and, pcnBg) were co-transfected by the calcium phosphate method into 293T cells (U.S. Patent No. 5,952,225), which express SV40 large T antigen. The producer cells were subsequently treated with 10 mM sodium butyrate for 8 to 12 hours to facilitate virion production, and retroviral supernatants were harvested at t=48 hours after transfection.

**Viral processing and incorporation of chimeric *env* proteins into retroviral vectors.** The level of expression of the nascent WT *env* proteins gp70 and/or the chimeric *env* "escort" proteins in 293T cell lysates was evaluated by Western analysis, using a rat monoclonal 83A25 antibody against the C-terminus of the SU domain of gp70, as previously described (39). To evaluate *env* incorporation into virions, viral particles were purified from soluble proteins and cell debris on a 20% sucrose gradient (in PBS), and the virion-associated proteins were subjected to Western analysis using anti-gp70 and anti-p30 antibodies (39).

**Determination of viral titers**. The infectious titers of test retroviral supernatants were standardized and quantified based on the expression of a nuclear-targeted β-galactosidase reporter gene (40), as determined by light microscopy. Briefly, 2.5 x 10⁴ NIH 3T3 cells in DMEM-10% FBS (D10) were plated in each well of 6-well plates one day prior to transduction. Medium was replaced with 1 ml of serial dilutions of the respective retroviral supernatant with 8 µg/ml polybrene for 2 hrs, after which time 1 ml of fresh D 10 medium was added to the cultures, which were maintained overnight at 37°C, 5% CO₂. The respective cultures were then stained with X-Gal histochemical stain 48 hrs after transduction to detect the presence of nuclear-targeted β-galactosidase in transduced cells (cells with blue-green nuclei). Viral titers were expressed as the number of β-galactosidase positive colony forming units per ml of vector supernatant (cfu/ml).

**Viral binding to human endothelial cells**. KSY1 Kaposi's human sarcoma cells (CRL-11448) and HUVE human umbilical cord vein endothelial cells (CC-2517) were obtained from the American Type Cell Culture Collection (ATCC, Bethesda Md) and Clonetics (San Diego, CA), respectively. For quantification of viral binding, 1 x 10⁶ KSY1 or HUVE cells were suspended in D10 in a microfuge tube, and were spun down for 15 sec, after which time 1 ml of test vector supernatant was added (viral titers were normalized at 1 x 10⁶ cfu/ml). The mixture was incubated with gentle shaking at RT for 30 min. The cells were washed twice with D10, and then resuspended in 300 µl in the presence of a rat monoclonal 83A25 Ab directed against the C-terminus of the gp70 MLV env protein (41) and incubated at RT for 1 hr. The cells were again washed twice with D10 medium, and then incubated in 500 µl 1:2500 HRP-goat anti-rat IgG (Zymed Laboratories Inc.) at RT for 30 min. After washing, the cells were incubated in 500 µl 1:1000 rat peroxidase anti-peroxidase antibody (Sternberger Monoclonals, Inc.) at RT for 30 min. After washing, the cells were resuspended in 100 µl TMB single solution (Zymed Laboratories Inc.), and transferred to a 96-well ELISA plate, where the intensity of the color reaction (blue) was read at OD650 nm on a Rainbow Spectra ELISA reader (TECAN US, Inc., NC).

**Transduction of human endothelial cells.** KSY1 or HUVE cells were cultured on 1% gelatin-coated dishes, in RPMI 1640 supplemented with either KSY1 medium consisting of 2% FCS, 1% sodium pyruvate, 1% essential amino acids, 1% non-essential amino acids, 1 mM glutamine, and 1% penicillin-streptomycin, or HUVE medium consisting of Iscove's modified Dulbeccos's/Ham F-12 media, supplemented with 15% FCS, 1% penicillin/streptomycin, 45 µg/ml heparin and 10 µg/ml ECGS. For transduction experiments, 05 to 1.0 x 10⁵ KSY1 or HUVE cells in 3 ml KSY1 medium or HUVE medium were plated into each gelatin-coated well in 6-well plates, and allowed to attach overnight at 37°C. The following morning, medium was replaced with 1 ml fresh KSY1 or HUVE medium. The cultures were transduced with 1 ml of each test vector supernatant normalized for equivalent viral titers in the presence of polybrene (8 µg/ml) at 37°C for 30 min. Thereafter, 2.5 ml fresh medium was added to the cultures which were then incubated overnight at 37°C. Medium was then replaced with fresh medium, and the cultures were further incubated at 37°C for another 48 hrs. The cells were then stained with X-Gal stain to visualize the presence of nuclear- targeted β-galactosidase activity under light microscopy. To quantify the resulting transduction efficiency, five low power (10X) fields of each test group were photographed (~1,500 cells per field) for KSY1 cells, and 10 low power fields (∼500 cells per field) for HUVE cells. Transduction efficiency was expressed as % by dividing the number of positive cells (cells with blue-staining nuclei) by the total number of cells per low power field X 100.

### Results

Six NGR-bearing TVTM motifs were selected for comparative evaluation (Figure 1). Two presumably cyclic peptides (TVTM-1 and TVTM-2) and one linear peptide (TVTM-3) previously demonstrated selectivity for tumor vasculature *in vivo* when expressed on the surface of filamentous phage (36). The remaining 3 TVTMs (TVTM-4, TVTM-5, and TVTM-6) are novel peptides. TVTM-4 introduces specific modifications of TVTM-3 including a hydrophobic residue (Leu) N-terminal and a polar residue (Ser) C-terminal to the core NGR motif. TVTM-5 and TVTM-6 constitute congeners of NGR motifs designed to examine the influence of adjacent C-terminal residues (Glu-Glu-Ser-Pro) present in the 9^{th} fibronectin Type III repeat (42). Glycine residues were included as linkers flanking each of the TVTMs in an effort to add flexibility to the secondary structures and facilitate folding of the chimeric retroviral envelope proteins. Each of the six TVTMs were encoded into cDNA sequences, prepared as double stranded oligodeoxynucleotides, and cloned into a modified CEEC vector (see Materials and Methods) in which a unique cloning site (specifically AvrII) has been added proximal to the proline-rich hinge region of the gp70 surface protein. The respective targeting ecotropic *env*, termed "escort" proteins, specifically designed to accompany the WT *env*, were prepared by ligation of the TVTM inserts into the BstEII and AvrII cloning sites, thus replacing the entire receptor binding domain of the ecotropic *env* protein with the respective TVTM/linker construct. Notably, the primary structure of the specified cloning sites within the CEE+ vector roughly approximates the primary structures flanking the core cell binding motifs (NGR and RGD) of the 9^{th} and 10^{th} Type III repeats found within fibronectin (see Figure 1).

Upon transient transfection, all of the six TVTM *env* proteins were expressed in human 293T retroviral vector producer cells, each exhibiting an apparent molecular mass of ~60 kDa. As seen in Figure 2 (panel A and C), the expression of the six *env* "escort" proteins was not impaired by co-expression of WT *env* proteins, which confer vector tropism and infectivity (ie., ecotropic, CEE or amphotropic, CAE). Each of the TVTM "escort" proteins could be detected in purified viral particles; however, notable differences in incorporation efficiencies were observed (see Figure 2, panels B and D). Five out of the six TVTM proteins were stably incorporated into viral particles in the absence of the WT CEE or CAE *env,* the exception being TVTM2, which was consistently lower in terms of incorporation efficiency. In the case of TVTM3 and TVTM6, co-expression of a wild-type envelope is seen to facilitate the incorporation of the modified "escort" protein, presumably due to structural complementation of the tertiary structures (43, 44). Remarkably, only the linear peptide sequences (TVTMs 3-6) were incorporated stoichiometrically with the WT env, which is indicative of proper assembly, processing, and stable incorporation into viral particles. Examination of corresponding viral titers on NIH 3T3 cells, which range from nil (for TVTM *env* alone) to 1.6 x 10⁸ for the TVTM2 + WT CEE vector (see Table 1), confirm that the observed fusion and infectivity is provided solely by the co-expression and fusogenic properties of the wild-type (CEE or CAE) envelope partner.

To examine the binding of TVTM-bearing retroviral vectors to activated endothelial cells *in vitro,* we utilized Kaposi's human sarcoma endothelial cells (KSY1, ATCC), which exhibit a constitutive (autocrine) expression of both VEGF and VEGF-receptors (45). The test vectors were co-expressed with ecotropic CEE (rodent-specific) envelope partners, which do not recognize/infect human cells. Figures 3A and 3B demonstrate high affinity binding of TVTM2, 4, 5 and 6 -bearing viral particles to KSY 1 cells compared to vectors bearing WT CEE *env* (p <.05), which was equal to or greater than that of the CAE (amphotropic) envelope-bearing (positive control) vectors (p <.05 for TVTM6 + CEE). In contrast, the cell binding affinities of TVTM1 and TVTM3 - bearing vectors were noted to be substantially lower than TVTM2,4,5 or 6-bearing vectors. Minimal binding was observed with the vector bearing only WT CEE (ecotropic) *env.*

Next, we examined the cell binding properties of TVTM-bearing retroviral vectors on normal human endothelial (HUVE) cells before and after pre-treatment with VEGF. As in the previous binding studies, the WT CEE *env* was co-expressed with the TVTM escort proteins instead of the WT CAE *env* to prevent vector fusion and entry, which would confound the interpretation of results. A marked increase in cell binding was provided by co-expression of the selected TVTM5 escort protein compared to the WT CEE env alone (p <.01) ; however, the observed increase in cell binding was reduced significantly by exposure of the HUVE cells to VEGF for 48 hours (p <.01). Additional studies were designed to examine the influence of cell density, as well as VEGF pre-treatment, on TVTM receptor expression in human endothelial cells. The results of these studies (see Figures 4A -4B) revealed a significant down-regulation of TVTM receptor expression on KSY1 cells exposed to VEGF when cultured under low density conditions (p <.05), and to a lesser degree in cells cultured at high densities. Similar results, obtained in HUVE cells (Figures 4C and 4D), demonstrate that TVTM receptor expression and/or binding to vectors displaying TVTM6 was highest in cells grown under low density conditions, which was again reduced by pre-treatment with VEGF (p <.001).

As in the cell binding studies described above, test vector supernatants were prepared for cellular transduction studies, with the exception that an amphotropic *env* partner (WT CAE) was utilized to enable the transduction of human cells. The resultant vectors were then normalized for equivalency of titer (1 x 10⁶ cfu/ml), based on the transduction of NIH 3T3 cells (see Table 1). Under these comparative conditions, vectors displaying TVTM escort proteins significantly enhanced transduction efficiency from approximately 122 ± SEM 1.4% for the WT CAE vector alone to 37.4 ± 1.7% for the WT CAE + TVTM5 vector (p< 0.001), and 31.0 ± 2.5% for the WT CAE + TVTM6 vector (p<0.001). Further, a ten-fold increase in transduction efficiency was observed in HUVE cells (0.4% for the WT CAE vector alone, to 4.2% for the WT CAE + TVTM-6 vector; p <.001; Table 2). These results demonstrate cell-specific targeting and transduction by enhancement of viral binding to an unidentified yet dynamic endothelial cell receptor for TVTMs.

### Discussion

Fibronectin, a ubiquitous adhesive glycoprotein found in relatively high concentrations in plasma as well as extracellular matrices (ECM), functions to mediate cell-ECM interactions during development, wound healing, and hemostasis. Soluble fibronectin is generally a dimer composed of two non-identical (alternatively spliced) subunits linked covalently by a pair of disulfide bonds, while the insoluble matrix form of fibronectin is arrayed as oligomers and fibrils (46). The primary structure of the fibronectin molecule is mosaic, consisting of a series of structurally distinct domains that interact with either ECM components or cell membrane receptors and are linked by flexible peptide segments (42, 46). Of particular interest is the central cell binding domain that is recognized by the integrin receptors of adherent cells (47, 48). Among the active sites that have been identified within these domains (49) include the RGD motif of the 10th type III repeat (50, 51) and the NGR motif of the 9^{th} type III repeat (33-35). While the binding of αᵥβ₁ and αᵥβ₃ integrins to RGD bearing proteins have been definitively shown (33-35,52), the cellular receptor(s) selective for the NGR cell binding motif remain to be identified (36). Extensive deletion studies of fibronectin have demonstrated the importance of the RGD cell binding domain within the 10^{th} type III repeat (53); however, additional domains contribute to the overall binding affinities, and a synergistic interaction specifically with adjacent type III repeats has been reported (54, 55). Filamentous phage displaying NGR motifs exhibit dissimilar integrin binding affinities and displacement kinetics *vis a vis* RGD motifs, indicating that the cellular receptor(s) for NGR motifs is (are) non-identical to that of RGD (35, 36). Moreover, the tumor homing ratio of the NGR-bearing phage in terms of adherence to tumor-associated vasculature versus normal vasculature *in vivo* is several fold greater than the RGD-bearing phage (36), thus confirming that the RGD and NGR cell binding motifs are indeed functionally distinct.

In this study, the performance of a series of NGR-bearing peptide congeners, termed tumor vasculature targeting motifs (TVTMs) was examined as a prelude to deploying retroviral vectors in pursuit of tumor vasculature and/or metastatic cancer. The TVTMs were displayed within the context of Moloney murine leukemia virus (MLV) env "escort" proteins, defined as non-infectious *env* proteins that accompany the infectious WT *env* to provide a gain-in-function-phenotype to the composite vector. In contrast to the cyclic configurations found to be advantageous when similar motifs were displayed within the context of either soluble peptides (56-58) or the surface proteins of filamentous phage (59,33-35), the cyclic NGR congeners (TVTM1 and TVTM2) were expressed readily in human producer cells (Figure 2A) but were either incorporated poorly into virions (TVTM2, see Figure 2B) or failed to exhibit the expected high affinity binding properties (TVTM1; Figure 3). For example, the cyclic TVTM2, which exhibits sequences flanking the core NGR motif that are very similar to TVTM5, was incorporated poorly into viral particles (see Figures 2B), but bound relatively well to target cells (Figures 3A and 3B). In contrast, the linear TVTMs were more readily incorporated into viral particles (Figure 2B & 2D), and, unlike free peptides or filamentous phage, the endothelial cell binding properties of chimeric vectors bearing linear TVTM designs were superior (Figure 3). Although general interference of the additional cysteine residues that generate the cyclic motifs with protein folding, disulfide bond formation, and secretion of the modified envelope proteins cannot be ruled out, it appears that the secondary and tertiary structures of the MLV envelope proteins may be more constrained than the filamentous phage or free peptides. In any event, because incorporation of retroviral envelope proteins into viral particles is an important feature of retroviral vector production, the linear peptide motifs are considered to be more favorable for this purpose.

The results of this study demonstrate that both addition and substitution of amino acid residues flanking the core NGR motif have profound effects on target cell binding. The cell binding affinity of TVTM4 is greater significantly than that of TVTM3, drawing attention first to the addition of a Ser residue immediately C-tenninal to the NGR motif, and second to the addition of a Leu residue immediately N-terminal. Interestingly, the C-terminal Ser residue is not present on the 9^{th} type III repeat (NGR) of fibronectin yet is evident from random alterations of the 10^{th} type III repeat (34, 35), and is conserved in a broad spectrum of proteins that have sequences similar to the RGD cell attachment-promoting sequence of fibronectin (50). The N-terminal Leu residue flanking the NGR motif of TVTM4, as well as TVTM5 and TVTM6, is found in the 9^{th} type III repeat of native fibronectin (Figure 1), and again in screens of random phage display libraries (34). TVTM5 and TVTM6 exhibit stable incorporation into retroviral particles and comparatively greater cell binding characteristics. These constructs represent linear peptides in which the flanking residues closely approximate the 9^{th} type III repeat of fibronectin, including (i) an N-terminal Ala-Leu leader sequence, (ii) - one (TVTM5) or two (TVTM6) negatively charged Glu residues C-terminal to the core NGR motif, followed by (iii) a Ser-Pro sequence, constituting what appears to be a type I Beta-turn (60) present in both the 9^{th} and 10^{th} type III repeat of native fibronectin.

The identification of the C-NGR-C motif (within the CNGRCVSGCAGRC phage) as the active site responsible for the greatest homing ratio (tumor/control organ) ascertained from random phage display libraries (36) is remarkable, considering the structure and function of the fibronectin cell-binding domain (48, 34). Conceptually, it is plausible that non-malignant but activated endothelial cells, under the influence of the tumor microenvironment, express an adhesion molecule and/or receptor complex that is not expressed highly on quiescent endothelial cells, and that this receptor recognizes a specific motif of fibronectin (i.e., NGR) that has been conserved and canalized by natural selection into a high affinity interaction. If, in fact, the functional selectivity lies in the expression of the undefined "TVTM receptor" and not in the exquisite sequence selectivity of the binding peptide (which is inherent within the primary structure of the ubiquitous fibronectin molecule) then it might well be advantageous to utilize fibronectin-like sequences *per se,* as in TVTM5 and TVTM6. Indeed, TVTMs 4, 5 and 6 are determined to be most favorable in terms of retroviral vector production, stability of membrane proteins in retroviral particles, and binding interactions of the resulting TVTM-bearing virions to proliferating endothelial cells and transformed KSY1 cells. In regard to the presumptive TVTM receptor, it is pertinent to note that pharmacological regulation of the cell binding properties of TVTM-bearing vectors was also observed in these studies (Figures 4A-D), as the TVTM binding properties of both normal and KSY1 endothelial cells in culture was highest in cells grown under low density conditions and was reduced significantly by prolonged (48 hour) pre-treatment with VEGF. The observed down-regulation of TVTM binding to target cells by VEGF, as well as increased cell density, suggests that other growth regulatory molecules may be implicated in the physiological up-regulation of the putative TVTM cellular receptor.

In terms of retroviral vector targeting and prospective gene therapy, the development of a targeted injectable vector which exhibits suitable affinities, selectivity, and stability for application *in vivo* remains a principal objective (61). Although a number of creative systems for targeting retroviral vectors have been designed (62), the most successful approach to date involves the insertion of a ligand that recognizes an ECM component to a portion of the MLV surface (SU) protein to concentrate the vector on the ECM in the vicinity of target cells (63). Similarly, co-localization of retroviral particles and target cells on specific fibronectin fragments has been shown to increase the transduction of cultured cells *in vitro* (64). Utilizing minimal/optimal peptide sequences, determined by random phage display technology to home specifically to tumor blood vessels, targeted anti-cancer drug-peptide conjugates were developed that exhibited enhanced efficacy and reduced toxicity when injected into the circulation of nude mice bearing human breast carcinoma xenografts (36). The present study expands our understanding of these tumor vascular targeting motifs (TVTMs) and their cognate receptors, and extends the potential utility of a defined subset of TVTMs to include targeted retroviral vectors.

In summary, an engineering approach to examine the performance of a designed series of NGR-bearing peptide congeners, including a series of linear peptides that approximate the 9^{th} type III repeat in fibronectin was utilized, and have determined that three novel designs (TVTM4. TVTM5 and TVTM6), presented in the context of MLV *env* "escort" proteins, including strategic linkers and cloning sites, are most suitable in terms of protein expression, retroviral vector production, and cell-binding affinities. These optimized TVTM-bearing *env* "escort" proteins were demonstrated further to function as targeting elements which served to increase the retroviral cell binding affinity and transduction efficiency in human endothelial cells, illustrating a potential utility for improving gene delivery in therapeutic angiogenesis and/or anti-angiogenesis/anti-cancer strategies.

**Table 1**

| **Viral Titers of Retroviral Vectors Displaying Tumor Vasculature Targeting Motifs** | | | | |
|---|---|---|---|---|
| Vector Name | Titer on NIH3T3 Cells cfu/ml | | Vector Name | Titer on NIH3T3 Cells cfu/ml |
| CEE | 1.7 x 10⁸ | | CAE | 5.6 x 10⁶ |
| CEE + TVTM1 | 1.7 x 10⁷ | | CAE + TVTM1 | 0.6 x 10⁶ |
| CEE + TVTM2 | 1.6 x 10⁸ | | CAE+TVTM2 | 1.5 x 10⁶ |
| CEE + TVTM3 | 5.8 x 10⁷ | | CAE+TVTM3 | 0.8 x 10⁶ |
| CEE + TVTM4 | 1.9 x 10⁷ | | CAE +TVTM4 | 0.6 x 10⁶ |
| CEE + TVTM5 | 2.5 x 10⁷ | | CAE + TVTM5 | 2.2 x 10⁶ |
| CEE + TVTM6 | 1.9 x 10⁷ | | CAE + TVTM6 | 2.5 x 10⁶ |

| | | | | |
|---|---|---|---|---|
| CEE = ecotropic retroviral vector bearing wild type envelope CEE + TVTM (1-6) = ecotropic retroviral vector displaying TVTMs (1-6) as escort *env* proteins CAE = amphotropic retroviral vector bearing wild type envelope CAE + TVTM (1-6) = amphotropic retroviral vector displaying TVTMs as escort env proteins | | | | |

Tabulated results are expressed as arithemetic means of duplicate determinations.

**Table 2**

| **Transduction Efficiency of Retroviral Vectors Displaying Tumor Vasculature Targeting Motifs** | | | | |
|---|---|---|---|---|
| Vector Name | Transduction Efficiency, % (in KSY-1 Cells) N=5 | | Vector Name | Transduction Efficiency, % (in HUVE Cells) N= 10 |
| CAE | 12.2 ± 1.4 | | CAE | 0.4 ± 0.1 |
| CAE + TVTM5 | 37.4 ± 1.7 (p <.001) | | CAE+TVTM5 | N.D. |
| CAE + TVTM6 | 31.0 ± 2.5 (p <.001) | | CAE + TVTM6 | 4.2 ± 0.5 (p <.001) |

| | | | | |
|---|---|---|---|---|
| CAE = amphotropic retroviral vector bearing wild type envelope CAE + TVTM5 = amphotropic retroviral vector displaying TVTM-5 as escort *env* protein CAE + TVTM6 = amphotropic retroviral vector displaying TVTM-6 as escort *env* protein Tabulated results are expressed as arithmetic mean ± S.E.M. N.D. = not determined p value = level of significance compared to CAE (vector bearing wild type envelope) | | | | |

### EXAMPLE 2

In the present Example, we incorporated selected TVTMs in a single amphotropic envelope configuration, and the resulting vectors were evaluated for their binding affinity to activated microvascular endothelial cells. Optimal TVTM-bearing vectors were further tested in transduction assays in vitro, for subsequent use in vivo in animal models of cancer.

### MATERIALS AND METHODS

Cell lines, cell culture conditions and plasmids. Murine Ras activated microvascular endothelial cells (SVR) were obtained from Clonetics (San Diego, CA, USA). Murine NIH3T3 (CRL 1658) and human umbilical vein endothelial (HUVE) cells (CRL 11268) were supplied by the American Type Culture Collection (Rockville, MD, USA). The human embryonic kidney cell line, transformed with SV40 large T antigen (293T) was kindly provided by Dr. Michele Calos, Stanford University, Palo Alto, CA. NIH 3T3, 293T and SVR cells were maintained in Dulbecco's modified Eagle's medium supplemented with 10% fetal bovine serum (D10; Gibco BRL, Gaithersburg, MD, USA).
HUVE cells were cultured on 1% gelatin-coated dishes, in RPMI 1640 supplemented with 10% fetal calf serum, 1% sodium pyruvate, 1% essential amino acids, 1% non-essential amino acids, 1 mM glutamine, and 1% penicillin-streptomycin. The plasmids pcgp containing the viral gag pol genes, and a retroviral vector, pcnBg, expressing a nuclear targeted β galactosidase construct were kindly provided by Drs. Paula Cannon and Ling Li respectively (USC Gene Therapy Laboratories, Los Angeles, CA). The rat monoclonal antibody, 83A25, directed against the C-terminus of the gp70 env protein was provided by Dr. Leonard Evans (Rocky Mountain Laboratories,MT).

**Molecular cloning of MLV-based amphotropic envelope proteins displaying tumor vasculature targeting motifs**. TVTM inserts with cohesive ends were cloned into a modified envelope construct (CAE-P) engineered to contain a unique pst1 restriction site near its N-terminus as described in the reference Hall et al., 2000 (66). Briefly, the MLV-based env construct was cut with Pst-1 and the linearized env plasmid was verified by restriction analysis on agarose gels and purified by the Gene Clean method (Bio 101, Vista, CA) prior to ligation with the respective TVTM insert and T4 DNA Ligase (New England Biolabs, Beverly, MA) for either 3 hours at room temperature or overnight at 4°C. In the resulting constructs, a TVTM peptide flanked by glycine linkers was inserted into the engineered Pst-1 site between a.a. 6 and 7 of the CAE-P envelope glycoprotein. After ligation, the various constructs of plasmid DNA were transformed into XL1 Blue strain of E. coli and grown on LB agar plates under ampicillin selection. Plasmid DNA was extracted from selected transformed clones using QIAprep Miniprep Kits (Qiagen, Valencia, CA). Each construct was confirmed by enzyme digestion and analysis of the respective inserts, followed by direct DNA sequence analysis.

**Generation of retroviral vector** stocks. Retroviral vectors bearing WT env and/or TVTM - bearing constructs were assembled using a three plasmid transient transfection system (38). The packaging components gag-pol, the WT env or a chimeric env, and a retroviral vector bearing a nuclear-targeted β-galactosidase expression construct expressed from CMV promoters were placed on separate plasmids, each containing the SV40 origin of replication. Ten µg of each plasmid (pCgp, pCAE, pTVTM-4, pTVTM-5 or pTVTM-6, and pCnBg) were co-transfected by the calcium phosphate method into 293T cells, which express SV40 large T antigen. The producer cells were subsequently treated with 10 mM sodium butyrate for 8 to 12 hours to facilitate virion production, and retroviral supernatants were harvested at t=24 hours after transfection.

**Viral processing and incorporation of chimeric env proteins into retroviral vectors**. The level of expression of the nascent WT env proteins gp70 and/or the chimeric env proteins in 293T cell lysates was evaluated by Western analysis, using a rat monoclonal 83A25 antibody against the C-terminus of the SU domain of gp70, as previously described (39). To evaluate env incorporation into virions, viral particles were purified from soluble proteins and cell debris on a 20% sucrose gradient (in PBS), and the virion-associated proteins were subjected to Western analysis using anti-gp70 and anti-p30 antibodies (39).

**Determination of viral titers**. Viral titers in murine NIH3T3 cells were determined as previously described, based on expression of the β-galactosidase transgene (40). Viral titer was expressed as number of β-galactosidase positive colony forming units (cfu)/ml medium (Table 3).

**Table 3**

| **Transduction Efficiency of Retroviral Vectors Displaying Tumor Vasculature Targeting Motifs** | | | | |
|---|---|---|---|---|
| Vector Name | Transduction Efficiency, % in SVR microvascular cells 30 min Incubation (n = 3) | | Vector Name | Transduction Efficiency, % in HUVE cells 30 min Incubation (n =3) |
| CAE | 0.9 ± 0.1 (10⁶ cfu/ml) | | CAE | 0.4 ± 0.1 (10⁶ cfu/ml) |
| | 14.7 ± 1.3 (10⁷ cfu/ml) | | | 0.9 + 0.3 (107 cfu/ml) |
| CAE+ TVTM-5 | 5.3 ± 0.6 (10⁶ cfu/ml) | | CAE + TVTM-5 | N.D. |
| CAE+ | 7.9 ± 1.2 (10⁶ cfu/ml) | | CAE + TVTM-6 | 4.2 ± 0.5 (10⁶ cfu/ml) |
| TVTM-6 | 27.6 ± 3.0 (10⁷ cfu/ml) | | | 8.3 ± 0.3 (10⁷ cfu/ml) |

| | | | | |
|---|---|---|---|---|
| CAE = amphotropic retroviral vector bearing wild type envelope CAE + TVTM-5 = amphotropic retroviral vector displaying TVTM-5 CAE + TVTM-6 = amphotropic retroviral vector displaying TVTM-6 Tabulated results are expressed as arithmetic mean + S.E.M. N.D. = not determined p value = level of significance compared to CAE (vector bearing wild type envelope) | | | | |

**Viral binding to human endothelial cells.** For quantification of viral binding, 5 x 106 HUVE cells were suspended in RPMI 1640 in a microfuge tube, and were spun down for 15 sec, after which time 1 ml of test vector supernatant was added (viral titers were normalized at ~1 x 106 cfu/ml). The mixture was incubated with gentle shaking at room temperature for 30 min. The cells were washed twice with D10 (DMEM-10%FBS) medium, and then resuspended in 300 µl in the presence of a rat monoclonal 83A25 Ab directed against the C-terminus of the gp70 MLV env protein (41) and incubated at room temperature for 1 hr. The cells were again washed twice with D10 medium, and then incubated in 500 µl 1:2500 HRP-goat anti-rat IgG (Zymed Laboratories Inc.) at RT for 30 min. After washing, the cells were incubated in 500 µl 1:1000 rat peroxidase anti-peroxidase antibody (Sternberger Monoclonals, Inc.) at room temperature for 30 min. After washing, the cells were resuspended in 100 µl TMBsingle solution (Zymed Laboratories Inc.), and transferred to a 96-well ELISA plate, where the intensity of the color reaction (blue) was read at OD650 nm on a Rainbow Spectra ELISA reader (TECAN US, Inc., NC).

**Transduction of activated microvascular endothelial (SVR) and HUVE cells.** For transduction experiments, 5 x 104 SVR cells in 3 ml D10 were plated into each uncoated plastic well, and 2.5 x 105 HUVE cells in 3 ml RPMI-2% (2%FBS in RPMI 1640) were plated into each gelatin-coated well, in 6-well plates, and allowed to attach overnight at 37oC. The cultures were transduced with 1 ml of each test vector supernatant normalized for equivalent viral titers in the presence of polybrene (8 µg/ml) at 37°C for 30 min (Liu et al., 2000). Thereafter, 2.5 ml of the respective fresh medium was added to the cultures which were then incubated for 48-72 hrs at 37°C. The cells were then stained with X-Gal stain to detect the presence of nuclear- targeted β- galactosidase activity under light microscopy. In triplicate experiments, the number of β-galactosidase positive cells (cells with blue-staining nuclei) were counted in three to four low power (10X) fields of each test group (~1,000 total SVR cells per field; -500 HUVE cells per field) and transduction efficiency was expressed as % β-galactosidase expressing cells.

### Results

Three NGR-bearing TVTM motifs were selected for comparative evaluation. TVTM-4 introduces specific modifications of TVTM-3 (65) including a hydrophobic residue (Leu) N-terminal and a polar residue (Ser) C-terminal to the core NGR motif. TVTM-5 and TVTM-6 constitute congeners of NGR motifs designed to examine the influence of adjacent C-terminal residues (Glu-Glu-Ser-Pro) present in the 9th fibronectin Type III repeat (42; 65). Glycine residues were included in the constructs as linkers flanking each of the TVTMs in an effort to add flexibility to the secondary structures and facilitate folding of the chimeric retroviral envelope proteins. Additional constructs incorporating 3 or 5 consecutive TVTM-6 were prepared for these comparative studies.

We have assessed comparative env protein expression levels of WT CAE and TVTM env proteins in 293T cell lysates. Retroviral supernatants were purified on a 20% sucrose gradient and the viral pellets were subjected to Western analysis using anti-gp70 and anti-p30 antibodies as described in Material and Methods of this Example. Upon transient transfection, all of the three TVTM envelope proteins were expressed and correctly processed in human 293T retroviral vector producer cells, each exhibiting an apparent molecular mass of -60 kDa as confirmed by Western analysis. The expression of the three modified envelope proteins was not impaired by the absence of the WT env protein and the levels of virion incorporation of retroviral vectors bearing WT CAE or chimeric TVTM env proteins were comparative. However, the incorporation of TVTM-4 into viral particles was appreciably less than TVTM-5 or TVTM-6. Viral titers of vectors displaying TVTM-5 and TVTM-6 ranged from 106 to 108, which indicate efficient assembly, processing, and stable incorporation into viral particles. Multiple TVTMs were also incorporated into virions without significant loss of titer.

To examine the binding of TVTM-bearing retroviral vectors to activated endothelial cells in vitro, we utilized proliferative human umbilical vein endothelial HUVE cells. The results of these binding studies demonstrate high affinity binding of TVTM-5 and TVTM-6-bearing viral particles to HUVE cells, which was significantly greater than that of the WT envelope-bearing (CAE) vectors. In contrast, the cell binding affinities of 3TVTM-6 and 5TVTM-6 bearing vectors were substantially lower, indicating that the presentation of multiple TVTM inserts does not enhance the performance of the fundamental targeting motif.

As in the cell binding studies described above, test vector supernatants were prepared for cellular transduction studies and were normalized for equivalency of titer based on the transduction of NIH 3T3 cells.

Under these comparative conditions, vectors displaying TVTMs significantly enhanced transduction efficiency in SVR cells compared to vectors bearing WT env. A parallel increase in transduction efficiency was also observed in HUVE cells (Table 3). These results demonstrate cell-specific targeting and transduction by enhancement of auxiliary viral binding to an unidentified yet dynamic endothelial cell receptor (36).

### Discussion

In Example 2, we constructed a series of modified 4070A amphotropic retroviral vectors incorporating TVTMs in a single envelope configuration, and demonstrated that the optimal constructs exhibited increased viral-cell binding and an 8-fold enhancement of transduction of activated microvascular endothelial cells under conditions (i.e., shortened incubation time and use of dilute non-concentrated vectors) that simulate the tumor microenvironment in vivo. One copy of TVTM-6 incorporated near the N-terminus of the amphotropic env was determined to be the optimal targeting motif in terms of retroviral vector production, envelope stability in retroviral particles, and binding interactions as well as transduction of activated microvascular endothelial cells.

In summary, we generated a retroviral vector displaying an optimal tumor vasculature targeting motif, that exhibits WT infectivity without co-expression of WT env, preferential endothelial cell binding, and enhanced transduction efficiency in activated microvascular endothelial cells in vitro. Taken together, the present vectors show great potential for further use in vivo as a targeted injectable vector.

### References

1. Dvorak, H.F., Brown, L.F., Detmar, M.,and Dvorak, A.M. Vascular permeability factor/vascular endothelial growth factor, microvascular hyperpermeability, and angiogenesis. Am. J. Pathol. *146*: 1029-1039, 1995.
2. Hanahan, D., and Folkman, J. Patterns and emerging mechanisms of the angiogenic switch during tumorigenesis. Cell *86*: 353-364,1996.
3. Burrows, FJ., and Thorpe, P.E. Vascular targeting: a new approach to the therapy of solid tumors. Pharmacol. Ther. *64*: 155-174, 1994.
4. Fidler IJ. Modulation of the organ microenvironment for treatment of cancer metastasis. J. Natl. Cancer Inst. *87*: 1588-1592, 1995.
5. Folkman, J. Antiangiogenic gene therapy. Proc. Natl. Acad. Sci. U.S.A. *95*: 9064-9066,1998.
6. Tanaka, T., Cao, Y., Folkman, J., and Fine, H.A. Viral vector-targeted antiagniogenic gene therapy utilizing an angiostatin complementary DNA. Cancer Res. *58*: 3362-3369, 1998.
7. Griscelli, F., Li, H., Bennaceur-Griscelli, A., Soria, J., Opolon, P., Soria, C., Perricaudet, M., Yeh, P., and Lu, H. Proc. Natl. Acad. Sci. U.S.A. *95*:6367-6372, 1998.
8. Bergers, G., Javaherian, K., Lo, K.M., Folkman, J., and Hanahan, D. Effects of angiogenesis inhibitors on multistage carcinogenesis in mice. Science *248:* 808-812, 1999.
9. Keck, PJ., Hauser, S.D., Krivi, G., Sanzo, K., Warren,T., Feder, J., and Connolly, D.T. Vascular permeability factor, an endothelial cell mitogen related to PDGF. Science *246:* 1309-1312, 1989.
10. Connolly, D.T., Heuvelman, D.M., Nelson, R., Olander, J.V., Eppley, B.L., Delfino, JJ., Siegel, N.R., Leimgruber, R.M., and Feder, J. Tumor vascular permeability factor stimulates endothelial cell growth and angiogenesis. J. Clin. Invest. *84*: 1470-1478, 1989.
11. Thomas, K.A. Vascular endothelial growth factor, a potent and selective angiogenic agent. J. BioL Chem. *271*: 602-606, 1996.
12. Senger, D.R., Galli, S.J., Dvorak, A.M., Perruzzi, C.A., Harvey, V.S., and Dvorak, H.F. Tumor cells secrete a vascular permeability factor that promotes accumulation of ascites fluid. Science *219*: 983-985, 1983.
13. Senger, D.R., Perruzzi, C.A., Feder, J., and Dvorak, H.F. A highly conserved vascular permeability factor secreted by a variety of human and rodent tumor cell lines. Cancer Res. *46*: 5629-5632, 1986.
14. Plate, K.H., Breier, G., Weich, H.A., Mennel, H.D., and Risau, W. Vascular endothelial growth factor and glioma angiogenesis: coordinate induction of VEGF receptors, distribution of VEGF protein and possible in vivo regulatory mechanisms. Int. J. Cancer *59*: 520-529, 1994.
15. Claffey, K.P., Brown, L.F., del Aguila, L.F., Tognazzi. K., Yeo, K.T., Manseau, EJ., and Dvorak, H.F. Expression of vascular permeability factor/vascular endothelial growth factor by melanoma cells increases tumor growth, angiogenesis, and experimental metastasis. Cancer Res. *56*: 172-181, 1996.
16. Brekken, R.A., Huang, X., King, S.W., and Thorpe, P.E. Vascular endothelial growth factor as a marker of tumor endothelium. Cancer Res. *58*: 1952-1959, 1998.
17. Mazure, NM., Chen, E.Y., Yeh, P., Laderoute, K.R., Giaccia, AJ. Oncogenic transformation and hypoxia synergistically act to modulate vascular endothelial growth factor expression. Cancer Res. *56*: 3436-3440, 1996.
18. Forsythe, J.A., Jiang, B.H., Iyer, N.V., Agani, F., Leung, S.W., Koos, R.D., and Semenza, G.L. Activation of vascular endothelial growth factor gene transcription by hypoxia- inducible factor 1. Mol. Cell Biol.*16*: 4604-4613, 1996.
19. Fukumura, D., Xavier, R., Sugiura, T., Chen, Y., Park, E., Lu, N., Selig, M., Neilsen, G., Taksir, T., Jain, R.K., and Seed, B. Tumor with VEGF promoter activity in stromal cells. Cell *94*: 715-725,1998.
20. Pepper, M.S., and Mandriota, SJ. Regulation of vascular endothelial growth factor receptor-2 (Flk-1) expression in vascular endothelial cells. Exp. Cell Res. *241*: 414-425,1998.
21. Waltenberger, J., Mayr, U., Pentz, S., and Hombach, V. Functional upregulation of the vascular endothelial growth factor receptor KDR by hypoxia. Circulation *94*: 1647-1654,1996.
22. Brogi, E., Schatteman, G., Wu, T., Kim, E.A., Varticovski, L., Keyt, B., Isner, J.M. Hypoxia-induced paracrine regulation of vascular endothelial growth factor receptor expression. J. Clin. Invest. *97*: 469-476, 1996.
23. Dvorak, H.F., Nagy, J.A., Dvorak, A.M. Structure of solid tumors and their vasculature: implications for therapy with monoclonal antibodies. Cancer Cells *3*: 77-85, 1991.
24. Ke-Lin, Q.H., Nagy, J.A., Eckelhoefer, I.A., Masse, E.M., Dvorak, A.M., and Dvorak, H.F. Vascular targeting of solid and ascites tumours with antibodies to vascular endothelial growth factor. Eur. J. Cancer *32A*: 2467-2473, 1996.
25. Brooks, P.C., Montgomery, A.M.P., Rosenfeld, M., Reisfeld, R.A., Hu, T., Klier, G., and Cheresh, D.A. Integrin αᵥβ₃ antagonists promote tumor regression by inducing apoptosis of angiogenic blood vessels. Cell *79*: 1157-1164, 1994.
26. Friedlander, M., Brooks, P.C., Shaffer, R.W., Kincaid, CM., Varner, J.A., and Cheresh, D.A. Definition of two angiogenic pathways by distinct alpha v integrins. Science *270*: 1500-1502, 1995.
27. Hammes, H.P., Brownlee, M., Jonczyk. A., Sutter, A., Preissner, K.T. Subcutaneous injection of a cyclic peptide antagonist of vitronectin receptor-type integrins inhibits retinal neovascularization. Nat. Med. *2*: 529-533, 1996.
28. Stromblad, S., and Cheresh, DA. Cell adhesion and angiogenesis. Trends Cell Biol. *6*:462-466,1996.
29. Adams, J.C., and Watt, F.M. Regulation of development and differentiation by the extracellular matrix. Development *117*: 1183-1198, 1993.
30. Lin, C.Q., and Bissell, M.J. Multi-faceted regulation of cell differentiation by extracellular matrix. FASEB J 7:737-743, 1993.
31. Johnson, R.C., Zhu, D., Augustin-Voss, H.G., and Pauli, B.U. Lung endothelial dipeptidyl peptidase IV is an adhesion molecule for lung-metastatic rat breast and prostate carcinoma cells. J. Cell BioL *121:* 1423-1432, 1993.
32. Pasqualini, R., and Ruoslahti, E. Organ targeting *in vivo* using phage display peptide libraries. Nature *380:* 364-366, 1996.
33. Koivunen, E., Gay, D.A., and Ruoslahti, E. Selection of peptides to the α₅β₁ integrin from phage display library. J. Biol. Chem. *268*: 20205-20210, 1993.
34. Koivunen, E., Wang, B., and Ruoslahti, E. Isolation of a highly specific ligand for the α₅β₁ integrin from a phage display library. J. Cell Biol.*124*: 373-379,1994.
35. Healy, J.M., Murayama, O., Maeda, T., Yoshino, K., Sekiguchi, K., and Kikuchi, M. Peptide ligands for integrin αᵥβ₃ selected from random phage display libraries. Biochemistry *34*: 3948-3955, 1995.
36. Arap, W., Pasquilani, R., and Ruoslahti, E. Cancer treatment by targeted drug delivery to tumor vasculature in a mouse model. Science *279*: 377-380, 1998.
37. Wu, B.W., Cannon, P.M., Gordon, E.M., Hall, F.L., and Anderson, W.F. Characterization of the proline-rich region of murine leukemia virus envelope protein. J. Virol. *72*: 5383-5391, 1998.
38. Soneoka, Y., Cannon, P.M., Ramsdale, E.E., Griffiths, J.C., Gaetano, R., Kingsman, S.M., and Kingsman, A.J. A transient three-plasmid expression system for the production of high titer retroviral vectors. Nucl. Acid Res. *23*: 628-633, 1995.
39. Zhu, N.L., Cannon, P.M., Chen, D., and Anderson, W.F. Mutational analysis of the fusion peptide of Moloney murine leukemia virus transmembrane protein p15E. J. Virol. *72*: 1632-1639, 1998.
40. Skotzko, M., Wu, L., Anderson, W.F., Gordon, E.M., and Hall, F.L. Retroviral Vector-mediated gene transfer of antisense cyclin G 1 (CYCG 1) inhibits proliferation of human osteogenic sarcoma cells. Cancer Res. 55: 5493-5498, 1995.
41. Evans, L.H., Morrison, F.G., Malik, J., Portis, J., Britt, WJ. A neutralizable epitope common to the envelope glycoprotein of ecotropic, polytropic, xenotropic, and amphotropic murine leukemia viruses. J. Virol. *64*: 6176-6183, 1990.
42. Komblihtt, A.R., Umezawa, K., Vibe-Pederson, K., and Barelle, F.E. Primary structure of human fibronectin: differential splicing may generate at least 10 polypeptides from a single gene. E.M.B.O. J. *4*:1755-1759,1985.
43. Zhao, Y., Lee, S., and Anderson, W.F. Functional interactions between monomers of the retroviral envelope protein complex. J. Virol. *71:* 6967-6972, 1997.
44. Anderson, W.F. Human gene therapy. Nature *392*: 25-33, 1998.
45. Masood, R., Cai, J., Zheng, T., Smith, D.L., Naidu, Y., and Gill, P.S. Vascular endothelial growth factor/vascular permeability factor is an autocrine growth factor for AIDS-Kaposi sarcoma. Proc. Natl. Acad. Sci. U.S.A. *94*:979-984, 1997.
46. Ayad, S., Boot-Handford, R.P., Humphries, M.J., Kadler, K.E., and Shuttleworth, C.A. The extracellular proteins. In The Extracellular Matrix Facts Book. Section II. 2^{nd} edition (Academic Press, Harcourt Brace & Co., Publishers, San Diego CA): 149-152, 1998.
47. Skorstengaard, K., Jensen, M.S., Sahl, O., Petersen, T.E., and Magnusson, S. Purification of a complete primary structure of the heparin-, cell-, and DNA-binding domain of bovine plasma fibronectin. Eur: J. Biochem. *161*: 441-453,1986.
48. Mohri, H. Fibronectin and integrins interactions. J. Invest. Med. *44*: 429-438, 1996.
49. Humphries, MJ., Komoriya, Akiyama, A., Olden, K., and Yamada, K.M. Identification of two distinct regions of the Type III connecting segment of human plasma fibronectin that promote cell type-specific adhesion. J. Biol. Chem. *262*: 6886-6892, 1987.
50. Pierschbacher, M.D., and Ruoslahti, E. Cell attachment activity of fibronectin can be duplicated by small synthetic fragments of the molecule. Nature *309:* 30-33, 1984.
51. Ruoshlahti, E., and Pierschbacher, M.D. Arg-Gly-Asp: a versatile cell recognition signal. Cell *44:* 5985-5988, 1986.
52. Koivunen, E., Wang, B., and Ruoshlati, E. Phage libraries displaying cyclic peptides with different ring sizes: ligand specificities of the RGD-directed integrins. Biotechnology *13*: 265-270, 1995.
53. Obara, M., Kang, M.S., and Yamada, K.M. Synthetic peptides competitively inhibit both direct binding to fibroblasts and functional biological assays for the purified cell-binding domain of human fibronectin:separable, synergistic sites mediate adhesive function. Cell *53*: 649-657, 1988.
54. Aota, S., Nagai, T., and Yamada, K.M. Characterization of regions of fibronectin besides the Arginine-Glycine-Aspartic acid sequence required for adhesive function of the cell-binding domain using site-directed mutagenesis. J. Biol. Chem. *266:* 15938-15943, 1991.
55. Nagai, T., Yamakawa, N., Aota, S., Yamada, S.S., Akiyama, S.K., Olden, K., and Yamada, K.M. Monoclonal antibody characterization of two distinct sites required for function of the central cell-binding domain of fibronectin in cell adhesion, cell migration, and matrix assembly. J. Cell Biol. *114*: 1295-1305, 1991.
56. Pierschbacher, M.D., and Ruoslahti, E. Influence of stereochemistry of the sequence Arg-Gly-Asp-Xa on binding specificity in cell adhesion. J. Biol. Chem.*262*: 17294-17298, 1987.
57. Gurrath, M., Muller, G., Kessler, H., Aumailley, M., and Timpl R. Conformation activity studies of rationally designed potent anti-adhesive RGD peptides. Eur. J. Biochem. *210*: 911-921, 1992.
58. Pfaff, M., Tangemann, K., Muller, B., Gurrath, M., Muller, G., Kessler, H., Timpl, R., and Engel, J. Selective recognition of cyclic RGD Peptides of NMR defined conformation by a_{π}β₃, αᵥβ₃, and α₅β₁, integrins. J. Biol. Chem. *269*:20233-20238, 1994.
59. O'Neil, K.T., Hoess, R.H., Jackson, S.A., Ramachandran, N.S., Mousa, S.A., and DeGrado, W.F. Identification of novel peptide antagonists for GPIIb/IIa from a conformationally constrained phage peptide library. Proteins *14*: 509-515, 1992.
60. Vulliet, P.R., Hall, F.L., Mitchell, J.P., and Hardie, D.G. A novel growth factor-sensitive protein kinase in pheochromocytoma. Proc. Western Pharmacol. Soc. *31:* 255-258,1988.
61. Anderson, W.F. Gene therapy. Sci. Amer. *273:* 124-128, 1995.
62. Cosset, F.L., Morling, FJ., Takeuchi, U., Weiss, R.A., Collins, M.K., and Russell, S.J. Retroviral retargeting by envelopes expressing an N-terminal binding domain. J. Virol. *69*:6314-22, 1995.
63. Hall, F.L., Gordon, E.M., Wu, L., Zhu, N.L., Skotzko, MJ., Starnes, V.A., and Anderson, W.F. Targeting retroviral vectors to vascular lesions by genetic engineering of the MoMuLV gp70 envelope protein. Hu Gene Therap 8: 2183-2192, 1997.
64. Hanenberg, H., Xiao, X.L., Dilloo, D., Hashino, K., Kato, I., and Williams, D.A. Colocalization of retrovirus and target cells on specific fibronectin fragments increases genetic transduction of mammalian cells. Nat. Med. 2: 876-882, 1996.
65. Liu L, Liu L, Anderson WF, Beart RW, Gordon EM, Hall FL. Incorporation of tumor vasculature targeting motifs into Moloney murine leukemia virus env escort proteins enhances retrovirus binding and transduction of human endothelial cells. J Virol 74:5320-5328, 2000.
66. Hall, F. L., Liu, L., Zhu, N.L., Stapfer, M., Anderson, W. F., Beart, R.W., and Gordon, E.M. Molecular engineering of matrix-targeted retroviral vectors incorporating a surveillance function inherent in von Willebrand factor. Hum. Gene Ther. 2000; 11: 983-993.

## Claims

1. A vector particle having a viral surface protein, wherein said viral surface protein includes TVTM-5 peptide having the sequence Ala Leu Asn Gly Arg Met Glu Ser Pro (SEQ ID NO. 5).

2. The vector particle of claim 1 wherein said vector particle is a retroviral vector particle and the modified viral surface protein is a modified retroviral envelope.

3. The retroviral vector particle of claim 2 wherein said retroviral envelope includes a receptor binding region, wherein said receptor binding region is modified to include the TVTM-5 peptide.

4. The vector particle of any of claims 1 to 3 wherein one copy of the TVTM-5 peptide is included in the viral surface protein.

5. The vector particle of any of claims 1 to 4 wherein the viral surface protein includes the TVTM-5 peptide near the N-terminus of the viral surface protein.

6. A modified polynucleotide encoding a modified viral surface protein, wherein said modified polynucleotide includes a polynucleotide encoding TVTM-5 peptide having the sequence Ala Leu Asn Gly Arg Met Glu Ser Pro (SEQ ID NO. 5).

7. The modified polynucleotide of claim 6 wherein said modified polynucleotide encodes a modified retroviral envelope polypeptide.

8. The modified polynucleotide of claim 7 wherein said retroviral envelope polypeptide includes a receptor binding region, wherein, in the modified polynucleotide, the polynucleotide encoding the receptor binding region is modified to include a polynucleotide encoding TVTM-5 peptide.

9. The modified polynucleotide of any of claims 6 to 8 wherein one copy of the polynucleotide encoding the TVTM-5 peptide is included in the modified polynucleotide encoding the viral surface protein.

10. The modified polynucleotide of any of claims 6 to 9 wherein the modified polynucleotide encoding the viral surface protein includes the polynucleotide encoding the TVTM-5 peptide near the 5'-terminus.

11. A producer cell for producing a retroviral vector particle having a modified envelope polypeptide, said producer cell including the modified polynucleotide of any of claims 6 to 10.

12. A peptide consisting of the sequence :
Ala Leu Asn Gly Arg Met Glu Ser Pro.

13. A polynucleotide encoding the peptide of Claim 12.

14. A proteoliposome including a wall, wherein said wall of said proteoliposome includes a modified retroviral envelope polypeptide including a TVTM-5 peptide having the sequence Ala Leu Asn Gly Arg Met Glu Ser Pro (Seq. ID NO: 5).

15. The vector particle of any of Claims 1 to 5, and further comprising a polynucleotide encoding an agent which is capable of inhibiting, preventing, or destroying the growth of cells of blood vessels of a tumor.

16. A medicament containing the vector particle of claim 15.

17. The use of the vector particle of Claim 15 in the manufacture of a medicament for treating a tumor.

18. The use of the vector particle of Claim 15 in the manufacture of a medicament useful to inhibit, prevent, or destroy the growth of cells of blood vessels of a tumor.

## Patentansprüche

1. Vektorpartikel mit einem viralen Oberflächenprotein, wobei das virale Oberflächenprotein ein TVTM-5 Peptid mit der Sequenz Ala Leu Asn Gly Arg Met Glu Ser Pro (SEQ ID NO. 5) aufweist.

2. Vektorpartikel nach Anspruch 1, wobei das Vektorpartikel ein retrovirales Vektorpartikel ist und das modifizierte virale Oberflächenprotein eine modifizierte retrovirale Hülle ist.

3. Retrovirales Vektorpartikel nach Anspruch 2, wobei die retrovirale Hülle eine Rezeptorbindungsregion aufweist, wobei die Rezeptorbindungsregion so modifiziert ist, dass sie das TVTM-5 Peptid umfasst.

4. Vektorpartikel nach einem der Ansprüche 1 bis 3, wobei eine Kopie des TVTM-5 Peptids im viralen Oberflächenprotein enthalten ist.

5. Vektorpartikel nach einem der Ansprüche 1 bis 4, wobei das virale Oberflächenprotein das TVTM-5 Peptid nahe dem N-Terminus des viralen Oberflächenproteins umfasst.

6. Modifiziertes Polynukleotid, das ein modifiziertes virales Oberflächenprotein kodiert, wobei das modifizierte Polynukleotid ein Polynukleotid umfasst, das das TVTM-5 Peptid mit der Sequenz Ala Leu Asn Gly Arg Met Glu Ser Pro (SEQ ID NO. 5) kodiert.

7. Modifiziertes Polynukleotid nach Anspruch 6, wobei das modifizierte Polynukleotid ein modifiziertes retrovirales Hüllpolypeptid kodiert.

8. Modifiziertes Polynukleotid nach Anspruch 7, wobei das retrovirale Hüllpolypeptid eine Rezeptorbindungsregion aufweist, wobei im modifizierten Polynukleotid das die Rezeptorbindungsregion kodierende Polynukleotid so modifiziert ist, dass es ein das TVTM-5 Peptid kodierendes Polynukleotid umfasst.

9. Modifiziertes Polynukleotid nach einem der Ansprüche 6 bis 8, wobei eine Kopie des das TVTM-5 Peptid kodierenden Polynukleotids im das virale Oberflächenprotein kodierenden Polynukleotid enthalten ist.

10. Modifiziertes Polynukleotid nach einem der Ansprüche 6 bis 9, wobei das das virale Oberflächenprotein kodierende, modifizierte Polynukleotid das das TVTM-5 Peptid kodierende Polynukleotid nahe dem 5'-Terminus aufweist.

11. Produktionszelle zum Herstellen eines retroviralen Vektorpartikels mit einem modifizierten Hüllpolypeptid, wobei die Produktionszelle das modifizierte Polynukleotid nach einem der Ansprüche 6 bis 10 aufweist.

12. Peptid bestehend aus der Sequenz:
Ala Leu Asn Gly Arg Met Glu Ser Pro.

13. Polynukleotid, das das Peptid nach Anspruch 12 kodiert.

14. Proteoliposom mit einer Wand, wobei die Wand des Proteoliposoms ein modifiziertes retrovirales Hüllpolypeptid umfasst, das ein TVTM-5 Peptid mit der Sequenz Ala Leu Asn Gly Arg Met Glu Ser Pro (SEQ ID NO. 5) enthält.

15. Vektorpartikel nach einem der Ansprüche 1 bis 5, und weiter umfassend ein Polynukleotid, das ein Agens kodiert, das das Wachstum von Blutgefäßzellen eines Tumors hemmen, verhindern oder unterdrücken kann.

16. Medikament, enthaltend das Vektorpartikel nach Anspruch 15.

17. Verwendung des Vektorpartikels nach Anspruch 15 zur Herstellung eines Medikaments zur Behandlung eines Tumors.

18. Verwendung des Vektorpartikels nach Anspruch 15 zur Herstellung eines Medikaments, das zur Hemmung, Verhinderung oder Unterdrückung des Wachstums von Blutgefäßzellen eines Tumors hilfreich ist.

## Revendications

1. Une particule vecteur ayant une protéine virale de surface; **caractérisée en ce que** ladite protéine virale de surface inclut le peptide TVTM-5 ayant la séquence Ala Leu Asn Gly Arg Met Glu Ser Pro (SEQ ID N°5).

2. Particule virale de la revendication 1 **caractérisée en ce que** ladite particule vecteur est une particule vecteur rétrovirale et **en ce que** la protéine virale de surface modifiée est une enveloppe rétrovirale modifiée.

3. Particule vecteur rétrovirale de la revendication 2 où ladite enveloppe rétrovirale inclut une région de liaison d'un récepteur, **caractérisée en ce que** ladite région de liaison d'un récepteur est modifiée pour inclure le peptide TVTM-5.

4. Particule vecteur de l'une quelconque des revendications 1 à 3, **caractérisée en ce qu'**une copie du peptide TVTM-5 est incluse dans la protéine virale de surface.

5. Particule vecteur de l'une quelconque des revendications 1 à 4, **caractérisée en ce que** la protéine virale de surface inclut le peptide TVTM-5 à proximité de l'extrémité N-terminale de la protéine virale de surface.

6. Polynucléotide modifié encodant une protéine virale de surface modifiée, **caractérisé en ce que** ledit polynucléotide modifié inclut un polynucléotide encodant un peptide TVTM-5 ayant la séquence Ala Leu Asn Gly Arg Met Glu Ser Pro (SEQ ID N°5).

7. Polynucléotide modifié de la revendication 6, **caractérisé en ce que** ledit polynucléotide modifié encode un polypeptide rétroviral d'enveloppe modifié.

8. Polynucléotide modifié de la revendication 7, où ledit polypeptide rétroviral d'-enveloppe inclut,une région de liaison d'un récepteur, **caractérisé en ce que** dans le polynucléotide modifié, le polynucléotide encodant la région de liaison d'un récepteur est modifié pour inclure un polynucléotide encodant le peptide TVTM-5.

9. Polynucléotide modifié de l'une quelconque des revendications 6 à 8, **caractérisé en ce qu'**une copie du polynucléotide encodant le peptide TVTM-5 est incluse dans le polynucléotide modifié encodant la protéine virale de surface.

10. Polynucléotide modifié de l'une quelconque des revendications 6 à 9, **caractérisé en ce que** le polynucléotide modifié encodant la protéine virale de surface inclut à proximité de l'extrémité 5' le polynucléotide encodant le peptide TVTM-5.

11. Cellule productrice pour la production d'une particule vecteur rétrovirale ayant un polypeptide d'enveloppe modifié, ladite cellule productrice incluant le polynucléotide modifié de l'une quelconque des revendications 6 à 10.

12. Peptide ayant la séquence : Ala Leu Asn Gly Arg Met Glu Ser Pro.

13. Polynucléotide encodant le peptide de la revendication 12.

14. Protéoliposome incluant une paroi, **caractérisé en ce que** ladite paroi dudit protéoliposome inclut un polypeptide rétroviral d'enveloppe modifié incluant un peptide TVTM-5 ayant la séquence Ala Leu Asn Gly Arg Met Glu Ser Pro (SEQ ID N°5).

15. Particule vecteur de l'une quelconque des revendications 1 à 5, et comprenant de plus un polynucléotide encodant un agent qui est capable d'inhiber, d'empêcher, ou de détruire la croissance des cellules de vaisseaux sanguins d'une tumeur.

16. Utilisation de la particule vecteur de la revendication 15 comme médicament.

17. Utilisation de la particule vecteur de la revendication 15 dans la fabrication d'un médicament pour le traitement d'une tumeur.

18. Utilisation de la particule vecteur de la revendication 15 dans la fabrication d'un médicament utile pour inhiber, empêcher ou détruire la croissance des cellules de vaisseaux sanguins d'une tumeur.


### SEQUENCE LISTING

(1) GENERAL INFORMATION:
   (i) APPLICANT: Anderson, W. French HaU, Frederick L. Gordon, Erlinda M.
   (ii) TITLE OF INVENTION**:** Modified Viral Surface Proteins which bind to cells of Tumor Vasculature
   (iii) NUMBER OF SEQUENCES: 10
   (iv) CORRESPONDENCE ADDRESS:
      (A) ADDRESSEE**:** Carella, Byme, Bain, Gilfillan, Cecchi, Stewart & Olstein
      (B) STREET: 6 Becker Farm Road
      (C) CITY: Roseland
      (D) STATE: New Jersey
      (E) COUNTRY: USA
      (F) ZIP: 07068
   (v) COMPUTER READABLE FORM:
      (A) MEDIUM TYPE: 3.5 inch diskette
      (B) COMPUTER: IBM PS/2
      (C) OPERATING SYSTEM: MS-DOS
      (D) SOFTWARE: Word Perfect 5.1
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER:
      (B) FILING DATE:
   (viii) ATTORNEY/AGENT INFORMATION:
      (A) NAME: Olstein, Elliot M.
      (B) REGISTRATION NUMBER: 24,025
      (C) REFERENCE/DOCKET NUMBER : 271010-451
   (ix) TELECOMMUNICATION INFORMATION:
      (A) TELEPHONE: 973-994-1700
      (B) TELEFAX: 973-994-1744
(2) INFORMATION FOR SEQ ID NO:1:
   (i) SEQUENCE CHARACTERISTICS:
      (A) LENGTH: 8 amino acids
      (B) TYPE: Amino Acid
      (C) STRANDEDNESS:
      (D) TOPOLOGY: linear
   (ii) MOLECULE TYPE: peptide
   (xi) SEQUENCE DESCRIPTION:SEQ ID NO:1:
   Ala Cys Asn Gly Arg Cys Val Ser
   5
(2) INFORMATION FOR SEQ ID NO:2:
   (i) SEQUENCE CHARACTERISTICS:
      (A) LENGTH: 10 amino acids
      (B) TYPE: Amino Acid
      (C) STRANDEDNESS:
      (D) TOPOLOGY: linear
   (ii) MOLECULE TYPE: peptide
   (xi) SEQUENCE DESCRIPTION:SEQ ID NO:2:
   Ala Cys Val Leu Asn Gly Arg Met Glu Cys
   5 10
(2) INFORMATION FOR SEQ ID NO:3:
   (i) SEQUENCE CHARACTERISTICS:
      (A) LENGTH: 7 amino acids
      (B) TYPE: Amino Acid
      (C) STRANDEDNESS:
      (D) TOPOLOGY: linear
   (ii) MOLECULE TYPE: peptide
   (xi) SEQUENCE DESCRIPTION:SEQ ID NO:3:
(2) INFORMATION FOR SEQ ID NO:4:
   (i) SEQUENCE CHARACTERISTICS:
      (A) LENGTH: 8 amino acids
      (B) TYPE: Amino Acid
      (C) STRANDEDNESS:
      (D) TOPOLOGY: linear
   (ii) MOLECULE TYPE: peptide
   (xi) SEQUENCE DESCRIPTION:SEQ ID NO:4:
   Ala Leu Asn Gly Arg Ser His Ala
   5
(2) INFORMATION FOR SEQ ID NO:5:
   (i) SEQUENCE CHARACTERISTICS :
      (A) LENGTH: 9 amino acids
      (B) TYPE: Amino Acid
      (C) STRANDEDNESS:
      (D) TOPOLOGY: linear
   (ii) MOLECULE TYPE: peptide
   (xi) SEQUENCE DESCRIPTION:SEQ ID NO:5:
   Ala Leu Asn Gly Arg Met Glu Ser Pro
   5
(2) INFORMATION FOR SEQ ID NO:6:
   (i) SEQUENCE CHARACTERISTICS:
      (A) LENGTH: 9 amino acids
      (B) TYPE: Amino Acid
      (C) STRANDEDNESS:
      (D) TOPOLOGY: linear
   (ii) MOLECULE TYPE: peptide
   (xi) SEQUENCE DESCRITPTION : SEQ ID NO:6:
   Ala Leu Asn Gly Arg Glu Glu Ser Pro
   5
(2) INFORMATION FOR SEQ ID NO: 7:
   (i) SEQUENCE CHARACTERISTICS:
      (A) LENGTH: 229 amino acids
      (B) TYPE: amino acid
      (C) STRANDEDNESS:
      (D) TOPOLOGY: linear
   (ii) MOLECULE TYPE: polypeptide
   (ix) FEATURE:
      (A) NAME/KEY: Receptor binding region of ecotropic gp70 protein
   (xi) SEQUENCE DESCRIPTION: SEQ ID NO:7:
   Ala Ser Pro Gly Ser Ser Pro
   5
   His Gln Val Tyr Asn Ile Thr Trp Glu Val
   10 15
   Thr Asn Gly Asp Arg Glu Thr Val Trp Ala
   20 25
   Thr Ser Gly Asn His Pro Leu Trp Thr Trp
   30 35
   Trp Pro Asp Leu Thr Pro Asp Leu Cys Met
   40 45
   Leu Ala His His Gly Pro Ser Tyr Trp Gly
   50 55
   Leu Glu Tyr Gln Ser Pro Phe Ser Ser Pro
   60 65
   Pro Gly Pro Pro Cys Cys Ser Gly Gly Ser
   70 75
   Ser Pro Gly Cys Ser Arg Asp Cys Glu Glu
   80 85
   Pro Leu Thr Ser Leu Thr Pro Arg Cys Asn
   90 95
   Thr Ala Trp Asn Arg Leu Lys Leu Asp Gln
   100 105
   Thr Thr His Lys Ser Asn Glu Gly Phe Tyr
   110 115
   Val Cys Pro Gly Pro His Arg Pro Arg Glu
   120 125
   Ser Lys Ser Cys Gly Gly Pro Asp Ser Phe
   130 135
   Tyr Cys Ala Tyr Trp Gly Cys Glu Thr Thr
   140 145
   Gly Arg Ala Tyr Trp Lys Pro Ser Ser Ser
   150 155
   Trp Asp Phe Ile Thr Val Asn Asn Asn Leu
   160 165
   Thr Ser Asp Gln Ala Val Gln Val Cys Lys
   170 175
   Asp Asn Lys Trp Cys Asn Pro Leu Val Ile
   180 185
   Arg Phe Thr Asp Ala Gly Arg Arg Val Thr
   190 195
   Ser Trp Thr Thr Gly His Tyr Trp Gly Leu
   200 205
   Arg Leu Tyr Val Ser Gly Gln Asp Pro Gly
   210 215
   Leu Thr Phe Gly Ile Arg Leu Arg Tyr Gln
   220 225
   Asn Leu
(2) INFORMATION FOR SEQ ID NO: 8:
   (i) SEQUENCE CHARACTERISTICS:
      (A) LENGTH: 60 amino acids
      (B) TYPE: amino acid
      (C) STRANDEDNESS:
      (D) TOPOLOGY: linear
   (ii) MOLECULE TYPE: polypeptide
   (ix) FEATURE:
      (A) NAME/KEY: Hypervariable polyproline region of amphotropic gp70 protein
   (xi) SEQUENCE DESCRIPTION : SEQ ID NO : 8:
   Gly Pro Arg Val Pro Ile Gly Pro Asn Pro
   5 10
   Val Leu Pro Asp Gln Arg Leu Pro Ser Ser
   15 20
   Pro Ile Glu Ile Val Pro Ala Pro Gln Pro
   25 30
   Pro Ser Pro Leu Asn Thr Ser Tyr Pro Pro
   35 40
   Ser Thr Thr Ser Thr Pro Ser Thr Ser Pro
   45 50
   Thr Ser Pro Ser Val Pro Gln Pro Pro Pro
   55 60
(2) INFORMATION FOR SEQ ID NO: 9:
   (i) SEQUENCE CHARACTERISTICS:
      (A) LENGTH: 687 bases
      (B) TYPE: nucleic acid
      (C) STRANDEDNESS: single
      (D) TOPOLOGY: linear
   (ii) MOLECULE TYPE: polynucleotide
   (ix) FEATURE:
      (A) NAME/KEY: polynucleotide encoding receptor binding region of ecotropic gp 70 protein
   (xi) SEQUENCE DESCRIPTION: SEQ ID NO: 9:
(2) INFORMATION FOR SEQ ID NO: 10:
   (i) SEQUENCE CHARACTERISTICS:
      (A) LENGTH: 180 bases
      (B) TYPE: nucleic acid
      (C) STRANDEDNESS: single
      (D) TOPOLOGY: linear
   (ii) MOLECULE TYPE: polynucleotide
   (ix) FEATURE:
      (A) NAME/KEY: polynucleotide encoding hypervariable polyproline region of amphotropic gp 70 protein
   (xi) SEQUENCE DESCRIPTION: SEQ ID NO: 10: